(19) Europäisches Patentamt
European Patent Office
Office européen des brevets

(11) **EP 2 700 038 B1**

(12) **EUROPEAN PATENT SPECIFICATION**

(45) Date of publication and mention
of the grant of the patent:
**21.04.2021 Bulletin 2021/16**

(51) Int Cl.:
**G06K 9/00** (2006.01)   **G06K 9/62** (2006.01)
**G01N 33/574** (2006.01)

(21) Application number: **12717662.6**

(22) Date of filing: **23.04.2012**

(86) International application number:
**PCT/EP2012/057387**

(87) International publication number:
**WO 2012/143556 (26.10.2012 Gazette 2012/43)**

(54) **ANALYZING THE EXPRESSION OF BIOMARKERS IN CELLS WITH MOMENTS**

ANALYSE DER EXPRESSION VON BIOMARKERN BEI ZELLEN MIT MOMENTEN

ANALYSE DE L'EXPRESSION DE BIOMARQUEURS DANS DES CELLULES AVEC DES MOMENTS

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **22.04.2011 US 201161478224 P**
**03.10.2011 US 201113252080**
**03.10.2011 US 201113252072**
**03.10.2011 US 201113252078**
**03.10.2011 US 201113252069**

(43) Date of publication of application:
**26.02.2014 Bulletin 2014/09**

(73) Proprietor: **Global Life Sciences Solutions USA LLC**
**Marlborough, MA 01752 (US)**

(72) Inventors:
• **MCCULLOCH, Colin, Craig**
  **Niskayuna, NY 12309 (US)**
• **GINTY, Fiona**
  **Niskayuna, NY 12309 (US)**
• **SEVINSKY, Christopher, James**
  **Niskayuna, NY 12309 (US)**

(74) Representative: **Brann AB**
**P.O. Box 3690**
**Drottninggatan 27**
**103 59 Stockholm (SE)**

(56) References cited:
| | |
|---|---|
| WO-A2-02/059377 | US-A1- 2004 121 413 |
| US-A1- 2008 118 934 | US-A1- 2010 184 093 |
| US-A1- 2011 091 081 | US-A1- 2011 091 081 |

US-A1- 2011 091 091

• ROST ET AL: "Microspectrofluorometry: errors, standardization and data processing", 1 August 1991 (1991-08-01), QUANTITATIVE FLUORESCENCE MICROSCOPY, CAMBRIDGE UNIVERSITY PRESS, GB, PAGE(S) 96 - 109, XP008156107, ISBN: 978-0-521-39422-2 page 109
• "12.1 (partially)" In: Rosenfeld, Azriel; Kak, Avinash: "Digital Picture Processing", 1982, Academic Press, New York, USA, XP002683408, vol. 2 page 286 - page 287 page 295
• QIN J ET AL: "Detection of citrus canker using hyperspectral reflectance imaging with spectral information divergence", JOURNAL OF FOOD ENGINEERING, BARKING, ESSEX, GB, vol. 93, no. 2, 1 July 2009 (2009-07-01), pages 183-191, XP026073106, ISSN: 0260-8774, DOI: 10.1016/J.JFOODENG.2009.01.014 [retrieved on 2009-01-30]
• CHEIN-I CHANG: "An Information-Theoretic Approach to Spectral Variability, Similarity, and Discrimination for Hyperspectral Image Analysis", IEEE TRANSACTIONS ON INFORMATION THEORY, IEEE PRESS, USA, vol. 46, no. 5, 1 August 2000 (2000-08-01), XP011027708, ISSN: 0018-9448, DOI: 10.1109/18.857802
• PATRICK C CHEN ET AL: "Segmentation by Texture Using a Co-Occurrence Matrix and a Split-and-Merge Algorithm", COMPUTER GRAPHICS AND IMAGE PROCESSING,, vol. 10, no. 2, 1 June 1979 (1979-06-01), pages 172-182, XP001376587, ISSN: 0146-664X

EP 2 700 038 B1

- M Sanchez-Carbayo: "Applying antibody arrays in cancer research", Microarrays, 1 November 2006 (2006-11-01), XP055058102, Retrieved from the Internet: URL:http://www.cli-online.com/fileadmin/ar timg/applying-antibody-arrays-in-cancer-re search.pdf [retrieved on 2013-03-28]
- BLOK L J ET AL: "Regulation of expression of Na+,K+-ATPase in androgen-dependent and androgen-independent prostate cancer", BRITISH JOURNAL OF CANCER, HARCOURT PUBLISHERS, vol. 81, no. 1, 1 January 1999 (1999-01-01), pages 28-36, XP002374011, ISSN: 0007-0920, DOI: 10.1038/SJ.BJC.6690647
- George Glass ET AL: "Simple: A Sequential Immunoperoxidase Labeling and Erasing Method", JOURNAL OF HISTOCHEMISTRY AND CYTOCHEMISTRY, vol. 57, no. 10, 13 April 2009 (2009-04-13), pages 899-905, XP055676909, US ISSN: 0022-1554, DOI: 10.1369/jhc.2009.953612

**Description**

FIELD

The invention relates generally to analyzing and visualizing the expression of biomarkers in individual cells, wherein the cells are examined in situ in their tissue of origin, to identify and understand patterns of expression that have an association with a diagnosis, a prognosis, or a response to treatment of a condition or a disease.

BACKGROUND

Examination of tissue specimens that have been treated to reveal the expression of biomarkers is a known tool for biological research and clinical studies. One such treatment involves the use of antibodies or antibody surrogates, such as antibody fragments, that are specific for the biomarkers, commonly proteins, of interest. Such antibodies or antibody surrogates can be directly or indirectly labeled with a moiety capable, under appropriate conditions, of generating a signal. For example, a fluorescent moiety can be attached to the antibody to interrogate the treated tissue for fluorescence. The signal obtained is commonly indicative of not only the presence but also the amount of biomarker present.

The techniques of tissue treatment and examination have been refined so that the level of expression of a given biomarker in a particular cell or even a compartment of the given cell such as the nucleus, cytoplasm or membrane can be quantitatively determined. The boundaries of these compartments or the cell as a whole are located using known histological stains. Commonly the treated tissue is examined with digital imaging and the level of different signals emanating from different biomarkers can consequently be readily quantified.

A technique has further been developed which allows testing a given tissue specimen for the expression of numerous biomarkers. Generally this technique involves staining the specimen with a fluorophore labeled probe to generate signal for one or more probe bound biomarkers, chemically bleaching these signals and re-staining the specimen to generate signals for some further biomarkers. The chemical bleaching step is convenient because there are only a limited number of signals that can be readily differentiated from each other so only a limited number of biomarkers can be examined in a particular step. But with bleaching, the sample may be re-probed and re-evaluated for multiple steps. This cycling method may be used on formalin fixed paraffin embedded tissue (FFPE) samples and cells. Digital images of the specimen are collected after each staining step. The successive images of such a specimen can conveniently be kept in registry using morphological features such as DAPI stained cell nuclei, the signal of which is not modified by the chemical bleaching method.

Another approach has been to examine frozen tissue specimens by staining them iteratively and photo bleaching the labels from the previous staining step before applying the next set of stains. The strength of the fluorescent signal associated with each biomarker evaluated is then extracted from the appropriate image.

There have been efforts to utilize this data to identify patterns of biomarker expression. One approach has been to look for such patterns in an entire tissue specimen and to binarize the fluorophore signals using a threshold values and generate various expression profiles that are then overlaid on an image of the tissue of interest.

U.S. Patent Application Publication Numbers US2011/0091081, entitled "Method and System for Analyzing the Expression of Biomarkers in Cells in Situ in Their Tissue of Origin," and U.S. Patent Application Publication Numbers US2011/0091091, entitled "Process and System for Analyzing the Expression of Biomarkers in Cells," both describe research and development work by General Electric prior to the present invention.

U.S. Patent Publication No. US2011/0091081 disclosed a process for acquiring data for analysis of the patterns of expression of multiple biomarkers in cells in their tissue of origin. The level of expression of multiple biomarkers in individual cells or in the subcellular compartments of the individual cells in situ in the tissue of origin of the cells was measured. The measurements could be conveniently made by treating the tissue specimens with antibodies or antibody surrogates specific to the biomarkers of interest. The antibodies or antibody surrogates were directly or indirectly labeled with moieties that give off optical signals when interrogated with light of the appropriate wavelength. The tissue specimens were repeatedly treated, with each treatment involving antibodies or antibody surrogates specific to different biomarkers than those involved in any other treatment and the signal generation from the immediately previous treatment was neutralized by optical or chemical means. The amount of each label bound to the biomarkers of interest by the antibodies or antibody surrogates was measured by subjecting the specimen to light of the appropriate wavelength and digitally imaging the response. The cells were conveniently segmented into individual cell units and their subcellular compartments (including membrane, cytoplasm and nucleus) were part of the data acquisition. The database stored the original measurement values and the location, cell or compartment of the cell, from which each measurement is drawn.

U.S. Patent Publication No. US2011/0091081 also disclosed a process for analyzing data representative of the patterns of expression of multiple biomarkers in cells in their tissue of origin. The numerical methods used to interrogate the database involved assigning certain attributes to each cell of interest based upon the measurements of biomarker expression levels and grouping those cells together which have similar biomarker expression attributes. The grouping involved an algorithm that groups together those cells which have a minimum distance between them in attribute space, i.e. two cells are included in the same group based on their distance from each other in n-dimensional space wherein each attribute is assigned a dimension.

U.S. Patent Publication No. US2011/0091081 further disclosed that groups of cells having similar patterns of expression of certain biomarkers could be a convenient basis for investigating associations between a biological condition and a given cell attribute. Each grouping could be examined to identify any cell attribute which is associated with the diagnoses or prognoses of a given condition or disease or with the response to a given therapy for a given condition or disease.

U.S. Patent Publication No. US2011/0091081 disclosed a process for displaying one or more groups of cells having similar patterns of expression of certain biomarkers. The groupings could be visualized by an overlay over one or more of the digital images of a field of view utilized to make the measurements of the levels of expression of the biomarkers. The overlay could show where in the original image cells occur which possess the profile of a given group. Images from different tissue specimens with such overlays could be compared to determine if the patterns of cells with one or more profiles, i.e. patterns of cells which belong to one or more groups, are indicative of any biological condition or process.

U.S. Patent Publication No. US2011/0091091 disclosed a process comprising measurement of the level of expression of multiple biomarkers in individual cells of a cellular sample, storing the measurement of biomarker expression of each cell as a data point in a database, and interrogating the database for data points having a similar pattern of biomarker expression using a computer algorithm where such similarity is determined by a numerical analysis that uses the level of expression of each biomarker as at least a semi-continuous variable. The data points with minimum variance were identified and grouped together. The group was assigned a new biomarker expression profile represented by a new data point, which is based on a central value for each attribute considered by the algorithm, thus forming a new data set. The steps were repeated with the new data set until a predetermined number of groups was generated.

U.S. Patent Publication No. US2011/0091091 also disclosed a method for using the grouping data for displaying a group of cells having similar patterns of expression of certain biomarkers. The method involved creating an image of one or more groups in a field of view of a cellular sample, by which each cell in a group was given a visible designation that they belong to the same group. The new image was registered to the original image of the sample to allow the images of the groups in a field of view to be sequentially overlaid and analyzed and displayed.

DESCRIPTION OF THE INVENTION

The present invention addresses one or more limitations of the prior art. For example, both U.S. Patent Publication No. US2011/0091081 and U.S. Patent Publication No. US2011/0091091 failed to disclose how to select an appropriate number of groups for a specific data set to investigate a possible association. U.S. Patent Publication No. US2011/0091091 discloses generating a predetermined number of groups within a specific data set, but does not disclose how to select the number of groups to generate. Without an approach for selecting appropriate number of groups for a specific data set, an appropriate number of groups may not be selected. Too few groups may result in cells with important distinctive characteristics being grouped together. An association of a subset of the grouped cells may be more difficult or impossible to identify. Too many groups will result in the need for unnecessarily complicated calculations and analysis. Too many groups may result in over-fitting the data set such that cells with no important distinctive characteristics are grouped separately. An association with two groups of cells that have no important distinctive characteristics may be more difficult or impossible to identify.

As another example, both U.S. Patent Publication No. US2011/0091081 and U.S. Patent Publication No. US2011/0091091 disclose limited techniques for displaying group-related information. Both publications disclose that the location of cells assigned to a group can be flagged within a much larger field of view. Both publications further disclose that cells within a much larger field of view can be flagged to indicate their assignment to one of a plurality of groups within the same view. Other than their relative location within a much larger field of view, however, such displays offers limited insight into the characteristics of cells within any particular group. Moreover, the groups resulting from multi-dimensional similarity grouping of cell may be inherently difficult for a medical practitioner to understand. Accordingly, embodiments taught herein involve distinct processes for analyzing a dataset.

Features, aspects, and advantages of the present invention will become better understood when the following description is read with reference to the accompanying, wherein:

FIG. 1 illustrates an exemplary computing environment suitable for practicing exemplary embodiments taught herein.

FIG. 2 illustrates an exemplary method of developing a model for identifying a predictive set of clusters of similar cells.

FIG. 3 illustrates an exemplary method of displaying cell cluster features.

FIG. 4 illustrates an exemplary method of applying a model set of clusters to new cell profile data.

FIG. 5 illustrates an exemplary method of developing a model for identifying a predictive set of moments of cell features from a data set.

FIG.6 illustrates an exemplary method of applying a model set of moments to new cell profile data.

FIG. 7 is a Receiver Operating Characteristic (ROC) curve for the cancer/normal classifier including first two moments of the marker data and the morphological features.

FIG. 8 is a ROC curve for the cancer only classifier including the first two moments of the marker data.

FIG. 9 is a variable importance plot for the cancer / normal classifier including first 2 moments of the marker data and the morphological features.

FIG. 10 is a variable importance plot for the cancer only classifier including the first two moments of the marker data.

FIG. 11 is a partial dependence plots for the top 4 features in the cancer / normal classifier.

FIG. 12 is a partial dependence plots for the top 4 features in the high-grade/low-grade classifier.

FIG. 13 is a graft showing the variable importance for survival model of whole cohort.

FIG. 14 is graphs of the partial dependence plots for survival model of whole cohort.

FIG. 15 is a graph showing variable importance for survival model on Gleason score > 0 cohort.

FIG. 16 is partial dependence plots for survival model of Gleason score > 0 cohort.

FIG. 17 is the observed average membrane P13Kp1 10a in invasive fields of view (FOVs) by batch.

FIG. 18 is the area under the ROC curve (AUC) for cancer/normal classifiers based on varying number of cell cluster features.

FIG. 19 is the area under the ROC curve for high grade/low grade cancer classifiers based on varying number of cell cluster features.

FIG. 20 is the ROC curve for the 20 cell cluster model of cancer/normal FOVs.

FIG. 21 is the ROC curve for the 20 ell cluster model of high grade/ low grade FOVs.

FIG. 22 is the variable importance for the 20 cluster classifier model of cancer/ normal FOVs

FIG. 23 is the variable importance of the 20 cluster classifier model of high grade/low grade cancer FOVs.

FIG. 24 is the partial dependence plots for the top 4 features in the cancer/normal classifier.

FIG. 25 is the partial dependence plots for the top 4 features in the high grade/low grade cancer classifier.

FIG. 26 is the observed FOV-level proportions of cluster 7 cells by batch (in each panel) and by cancer vs. normal (labeled true/false). The x-axis is the square root of the cluster 7 proportion in the FOV.

FIG. 27 is the signature for cluster 7 of 20. The ball end is of each horizontal line is the average in cluster 7; the other end is the average of all 20 clusters.

FIG. 28 is the performance metrics for survival models on the whole cohort. RSF concordance and AUC for classifying death of prostate cancer within 3, 5, and 10 years. The performance of the null model including only age and Gleason score is shown as a horizontal line.

FIG. 29 is the performance metrics for survival models on the Gleason score >0 cohort. RSF concordance and AUC for classifying death of prostate cancer within 3, 5, and 10 years. The performance of the null model including only age and Gleason score is shown as a horizontal black line.

FIG. 30 is the variable importance for the survival model of the whole cohort.

FIG. 31 is the partial dependence plots for the top four features in the whole cohort survival analysis.

FIG. 32 is the variable importance of the survival model on the Gleason score > 0 cohort.

FIG. 33 is the partial dependence of the top four features in the 20 cluster model of the Gleason score > 0 cohort.

FIG. 34 is the signatures of Clusters 6/6 and 1/20, both indications of shorter survival time.

FIG. 35 illustrates exemplary montages of two cells in a cluster.

Embodiments taught herein leverage multiplexed biometric images that are generated through known techniques, such as through a multiplexing staining-destaining technique. The images illustrate the expression of biomarkers within individual cells that enables comparison of the individual cells to each other. The individual cells are part of a larger cell sample. For example, the cell sample may be a group of cells from a cell culture, a tissue sample, organ, tumor, or lesion. The individual cells may also be part of a group of specimens of similar tissue from different subjects. These groups of cells may represent one or more disease or condition models, different stages within a disease or condition model, or one or more responses to treatment of a disease or condition.

Images of each stained field of view are generated through known techniques, such as with a digital camera coupled with an appropriate microscope and appropriate quality control routines. Automated image registration and analysis may also be used to quantify the biomarker concentration levels for individual delineated cells, or even subcellular compartments, such as nucleus, cytoplasm, and membrane. The data values resulting from the multiplexing and image analysis of cells may be stored alone or in conjunction with data that is the result of further analysis. The database preserves the identity of the measurement of strength of the biomarker expression including the tissue and the location within the tissue from which it was drawn. The location should include the particular cell from which a particular measurement was drawn and may also include the compartment, nucleus, cytoplasm or membrane, associated with the measurement. The information is stored in a database which may be maintained in a storage device 116 or in a network device 126.

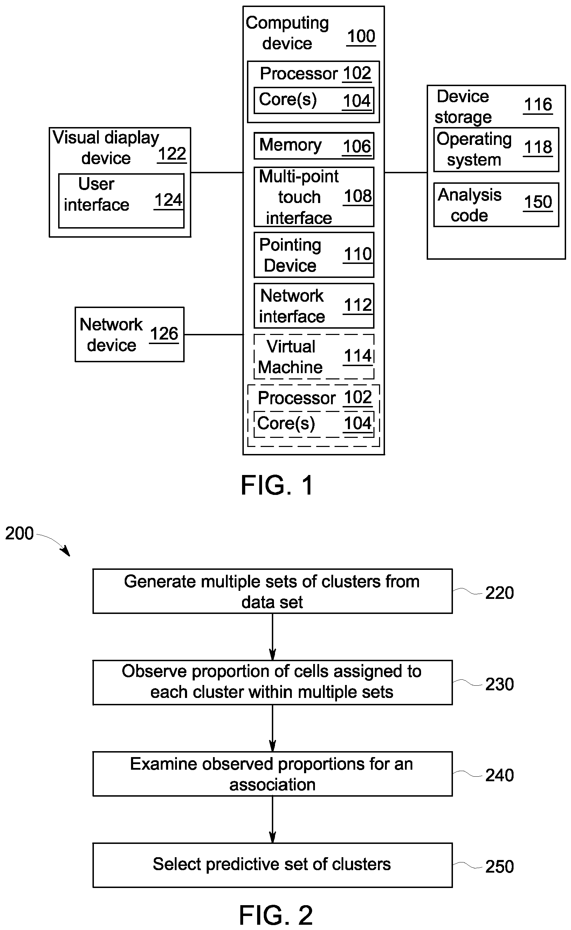

FIG. 1 illustrates an exemplary computing environment suitable for practicing exemplary embodiments taught herein. The environment includes a computing device 100 with associated peripheral devices. Computing device 100 is programmable to implement executable code 150 for various methods as taught herein. Computing device 100 includes a storage device 116, such as a hard-drive, CD-ROM, or other non-transitory computer readable media. Storage device 116 stores an operating system 118 and other related software. Computing device 100 may further include memory 106. Memory 106 may comprise a computer system memory or random access memory, such as DRAM, SRAM, EDO RAM, etc. Memory 106 may comprise other types of memory as well, or combinations thereof. Computing device 100 may store, in storage device 116 and/or memory 106, instructions for implementing and processing every module of the executable code 150.

Computing device 100 also includes processor 102 and, one or more processor(s) 102' for executing software stored in the memory 106, and other programs for controlling system hardware. Processor 102 and processor(s) 102' each can be a single core processor or multiple core (104 and 104') processor. Virtualization may be employed in computing device 100 so that infrastructure and resources in the computing device can be shared dynamically. Virtualized processors may also be used with executable analysis code 150 and other software in storage device 116. A virtual machine 114 may be provided to handle a process running on multiple processors so that the process appears to be using only one computing resource rather than multiple. Multiple virtual machines can also be used with one processor.

A user may interact with computing device 100 through a visual display device 122, such as a computer monitor, which may display the user interfaces 124 or any other interface. The visual display device 122 may also display other aspects or elements of exemplary embodiments, e.g. an icon for storage device 116. Computing device 100 may include other I/O devices such a keyboard or a multi-point touch interface 108 and a pointing device 110, for example a mouse, for receiving input from a user. The keyboard 108 and the pointing device 110 may be connected to the visual display device 122. Computing device 100 may include other suitable conventional I/O peripherals.

Computing device 100 may include a network interface 112 to interface with a network device 126 via a Local Area Network (LAN), Wide Area Network (WAN) or the Internet through a variety of connections including, but not limited to, standard telephone lines, LAN or WAN links (e.g., 802.11, T1, T3, 56kb, X.25), broadband connections (e.g., ISDN, Frame Relay, ATM), wireless connections, controller area network (CAN), or some combination of any or all of the above. The network interface 112 may comprise a built-in network adapter, network interface card, PCMCIA network card, card bus network adapter, wireless network adapter, USB network adapter, modem or any other device suitable for enabling computing device 100 to interface with any type of network capable of communication and performing the operations described herein.

Moreover, computing device 100 may be any computer system such as a workstation, desktop computer, server, laptop, handheld computer or other form of computing or telecommunications device that is capable of communication and that has sufficient processor power and memory capacity to perform the operations described herein.

Computing device 100 can be running any operating system 118 such as any of the versions of the Microsoft® Windows® operating systems, the different releases of the Unix and Linux operating systems, any version of the MacOS® for Macintosh computers, any embedded operating system, any real-time operating system, any open source operating system, any proprietary operating system, any operating systems for mobile computing devices, or any other operating system capable of running on the computing device and performing the operations described herein. The operating system may be running in native mode or emulated mode.

FIG. 2 illustrates a method 200 of developing a model for identifying a predictive set of clusters of similar cells from a data set. The method leverages a data set that may be stored, for example, in storage device 116 or network device 126. The data set comprises cell profile data. The cell profile data includes multiplexed biometric images capturing the expression of a plurality of biomarkers with respect to a plurality of fields of view in which individual cells are delineated and segmenting into compartments. The cell profile data is generated from a plurality of tissue samples drawn from a cohort of patients having a commonality. The commonality may be, for example, that the patients share a disease or condition. Alternatively, the commonality may be, for example, that the patients share a preliminary diagnosis of the same disease or condition. The data set further comprises an association of the cell profile data with at least one piece of meta-information including a field of view level assessment or a patient-level assessment related to the commonality. The patient-level assessment may be, for example, survival time after surgery.

In 220, a plurality of sets of clusters of similar cells are generated from the data set. One or more processors, such as processors 102, 102', generate the plurality of sets of clusters. Each of the plurality of sets of clusters generated comprises a unique number of clusters. Each cell is assigned to a single cluster in each of the plurality of sets of clusters. Each of the plurality of clusters in each of the plurality of sets of clusters comprises cells having a plurality of selected attributes more similar to the plurality of selected attributes of other cells in that cluster than to the plurality of selected attributes of cells in other clusters in the set.

Cell similarity is determined at least in part from a comparison of at least one attribute of a cell based on the expression of at least one of the plurality of biomarkers. A cell attribute used for cluster generation in method 200 is a nucleus intensity ratio defined by subtracting half of the sum of the median intensity of the membrane and the median intensity of the cytoplasm from the median intensity of the cell nucleus's expression of at least one of the plurality of biomarkers. A cell attribute used for cluster generation in method 200 is a membrane intensity ratio defined by subtracting half of the sum of the median intensity of the nucleus and the median intensity of the cytoplasm from the median intensity of the cell membrane's expression of at least one of the plurality of biomarkers. A cell attribute used for cluster generation in method 200 is a cytoplasm intensity ratio defined by subtracting half of the sum of the median intensity of the membrane and the median intensity of the nucleus from the median intensity of the cell cytoplasm's expression of at least one of the plurality of biomarkers. A cell attribute used for cluster generation in method 200 is a median intensity of the whole cell. For example, the nucleus intensity ratio for each of the plurality of biomarkers may be the basis for generating sets of clusters.

Some embodiments of method 200, not covered by the claimed invention, determine cell similarity at least in part from a comparison of two attributes of a cell based on the expression of at least one of the plurality of biomarkers. For example, a nucleus intensity ratio and a membrane intensity ratio for at least one of the plurality of biomarkers may be a basis for generating sets of clusters. Some embodiments of method 200, not covered by the claimed invention, determine cell similarity at least in part on a comparison of three attributes of a cell based on the expression of at least one of the plurality of biomarkers. For example, a nucleus intensity ratio, a membrane intensity ratio, and a cytoplasm intensity ratio for at least one of the plurality of biomarkers may be a basis for generating sets of clusters. Some embodiments of method 200 determine cell similarity at least in part on a comparison of four attributes of a cell based on the expression of at least one of the plurality of biomarkers. For example a nucleus intensity ratio, a membrane intensity ratio, a cytoplasm intensity ratio, and a median intensity of the whole cell for at least one of the plurality of biomarkers may be a basis for generating sets of clusters. Embodiments of method 200, not covered by the claimed invention, determine cell similarity from other combinations of attributes. Some embodiments of method 200, not covered by the claimed invention, determine cell similarity from a comparison of more than four attributes of a cell based on the expression of at least one of the plurality of biomarkers.

Some embodiments of method 200, not covered by the claimed invention, generate clusters of the similarity of cells by applying a K-medians clustering algorithm to the relevant set of cell attributes. Other embodiments of method 200, not covered by the claimed invention, generate clusters of the similarity of cells by applying a K-mean clustering algorithm to the relevant set of cell attributes. In some embodiments, not covered by the claimed invention, analysis code 150 includes the clustering algorithm.

The plurality of sets of clusters is generated from a normalized data set. Some embodiments, not covered by the claimed invention, may normalize the measurement values to determine the mean and standard deviation of all the measurements associated with a given biomarker in a given study and subtract this mean value from each measurement value and then to divide the resultant difference by the standard deviation. In some embodiments, not covered by the claimed invention, the measurement values are expressed on a log scale of the intensity of the expression of a biomarker in the image. A subtraction in measurement values expressed in the log scale may correspond to a division in the original raw measurement scale. Other embodiments, not covered by the claimed invention, may normalize the measurement values to determine the median intensity of a whole cell's expression for all cells within a batch of measurements and subtract this median value from each measurement value in the batch. Such median intensity may apply to the expression of a specific biomarker. This normalized or standardized value may be stored in the database or generated as part of the processing of the data set in the database.

The plurality of sets of clusters in some embodiments, not covered by the claimed invention, is generated from a filtered data set. Such filtering may be done as a quality control measure. Such filtering may exclude, for example, cell profile data related to cells comprising at least one compartment represented by fewer than a threshold number of pixels in the multiplexed image. Filtering may also be done for reasons beyond quality control. Such filtering may exclude, for example, cell profile data related to normal cells from the data set used to generate the plurality of sets of clusters of similar cells.

In 230, a proportion of the cells assigned to each cluster within each of the plurality of sets of clusters is observed. In 240, the observed proportions are examined for an association with the at least one piece of meta-information including the field of view level assessment or the patient-level assessment related to the commonality. An association between observed proportions and a field of view level assessment or a patient-level assessment can be derived by fitting a classification model with the assessment as the outcome and proportions of observed clusters as the predictors. Several classification analysis frameworks exist, including random forests, neural networks, and logistic regression. For example, an association between tissue grade and presence and number of cells observed from a given cell cluster is derived, in some embodiments not covered by the claimed invention, by fitting a random forest classification model with tissue grade as the outcome and proportions of observed clusters as the predictors. An association between tissue grade and presence and number of cells observed from a given cell cluster is derived, in other embodiments not covered by the claimed invention, by fitting a neural network classification model with tissue grade as the outcome and proportions of observed clusters as the predictors. Some embodiments of method 200, not covered by the claimed invention, further comprise examining the observed proportions in the selected set of clusters for a univariate association with an assessment. Other embodiments of method 200, not covered by the claimed invention, further comprise examining the observed proportions in the selected set of clusters for a multivariate association with an assessment.

In some embodiments of method 200, not covered by the claimed invention, the observed proportion of cells is the observed proportion of the cells of each field of view assigned to each cluster. In these embodiments, not covered by the claimed invention, the observed proportions are examined for an association with the field of view level assessment related to the commonality; and a predictive set of clusters is selected through on a comparison of the performance of the field of view level assessment models based on the plurality of sets of clusters.

In some embodiments of method 200, not covered by the claimed invention, the observed proportion of cells is the observed proportion of the cells of each patient assigned to each cluster. In these embodiments, not covered by the claimed invention, the observed proportions are examined for an association with a prognosis of a condition or a disease and a plurality of sets of clusters is selected through on a comparison of a performance of a patient level assessment model based on the plurality of sets of clusters.

In some embodiments, not covered by the claimed invention, the assessments are grouped. In cohorts of prostate cancer patients, for example, assessments resulting in a Gleason score of 2 or 3 may be grouped together. In these embodiments, not covered by the claimed invention, the plurality of sets of clusters are examined for an association with the grouped assessments related to the commonality of the patient cohorts. For example, combinations of attributes can be examined for an association with a low Gleason score where samples having a Gleason score of 2 or 3 are grouped together. Field of view level assessments of cohorts of other types of cancer may involve assessments of other types of tumors having their own relevant tumor grades. Other cancer grading systems include, for example, the Bloom-Richardson system for breast cancer and the Fuhrman system for kidney cancer. Whenever cancer or other diseases have assessments that may fall within more than two grades or categories, similar grades or categories may be grouped in some embodiments, not covered by the claimed invention.

In 250, one of the plurality of sets of clusters is selected based on a comparison of the performance of at least one model of the plurality of sets of clusters. In some embodiments not covered by the claimed invention, visual display device 122 enables the selection to be made. Similar classification models can be created for each of the plurality of sets of clusters. In some embodiments, not covered by the claimed invention, one or more processors, such as processors 102, 102', create the classification models. Each model predicts an assessment based on cell cluster proportions in the corresponding set of clusters. In some embodiments, not covered by the claimed invention, each model predicts tissue grade based on cell cluster proportions in the corresponding set of clusters. The performance of the model of each set of clusters can be evaluated by various metrics of predictive performance in a test set of data not used for developing the model. Performance metrics that can be used to compare the sets of clusters based on the models include sensitivity, specificity, area under the receiver operating characteristic curve (also called concordance). The set of clusters to be used may then be selected based on one or more of the model performance metrics. For example, in some embodiments, not covered by the claimed invention, the set of clusters associated with the highest concordance is selected. In other embodiments, not covered by the claimed invention, the set of clusters associated with the highest concordance is not selected due to apparent over-fitting of the data. The selected set comprising a predictive set of clusters. Some embodiments of method 200, not covered by the claimed invention, further comprise comparing the performance of at least one model with respect to the number of clusters in each of the plurality of sets of clusters.

Some embodiments of method 200, not covered by the claimed invention, further comprise selecting a set of clusters having a number of clusters below which a greater number of clusters in the set of cluster provides a decrease in performance. Some embodiments of method 200, not covered by the claimed invention, further comprise selecting a set of clusters having a number of clusters above which a greater number of clusters in the set of cluster does not offer a statistically significant increase in performance. Some embodiments of method 200, not covered by the claimed invention, further comprise selecting a set of clusters based on a performance of the at least one model of the set of clusters corresponding to a performance metric greater than a pre-defined threshold, which may be for example a concordance of 0.85 or greater. Some embodiments of method 200, not covered by the claimed invention, further comprise identifying at least one predictive cluster from the predictive set of clusters.

Some embodiments of method 200, not covered by the claimed invention, divide the cell data into training data and test data, generate the plurality of sets of clusters of similar cells from training data, and determine the performance of the at least one model from the testing data.

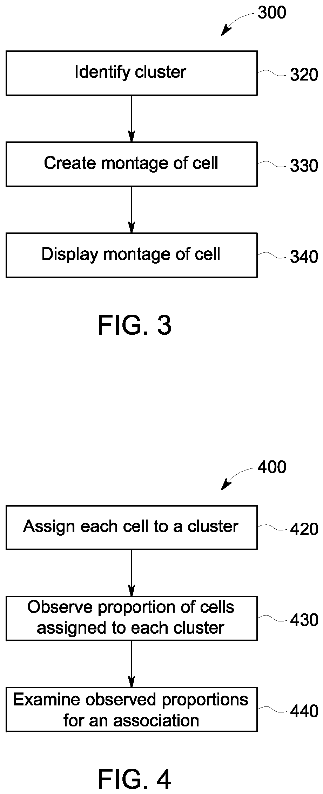

FIG. 3 illustrates an exemplary method 300 of displaying cell cluster features. The method leverages a data set that may be stored, for example, in storage device 116 or network device 126. The data set comprises cell profile data. The cell profile data includes multiplexed biometric images capturing the expression of a plurality of biomarkers with respect to a plurality of fields of view in which individual cells are delineated and segmenting into compartments.

In 320, a first cluster in a plurality of clusters of similar cells from the data set is identified. Each cell is assigned to one of the plurality of clusters. Each cluster in the plurality of clusters includes cells having a plurality of selected attributes more similar to the plurality of selected attributes of other cells in that cluster than to the plurality of selected attributes of cells in other clusters in the set. Cell similarity may be judged and clustering may done by any of the techniques discussed above with respect to 220.

In 330, a montage of a first cell in the first cluster is created. One or more processors, such as processors 102, 102', create the montage. The montage comprises a portion of at least some multiplexed images describing the first cell's expression of each of a plurality of biomarkers. Each portion of the at least some images includes the first cell and a small region of interest around the first cell.

In 340, the montage of the first cell in the first cluster is displayed to enable a user to understand a feature of the first cluster. The montage is displayed on visual display device 122. The montage of the first cell displayed in some embodiments of method 300, not covered by the claimed invention, comprises a series of juxtaposed portions of the at least some images of a field of view describing the first cell's expression of each of a plurality of biomarkers. The montage of the first cell displayed in other embodiments of method 300, not covered by the claimed invention, comprises a series of superimposed portions of the at least some images of a field of view describing the first cell's expression of each of a plurality of biomarkers.

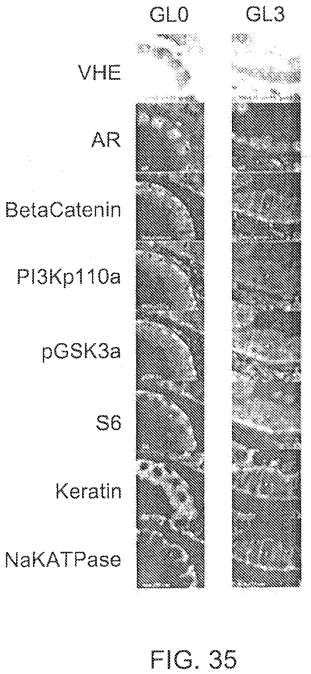

Some embodiments of method 300, not covered by the claimed invention, further include creating and displaying a montage of a second cell in the first cluster. The montage of the second cell comprises a portion of at least some images of a field of view describing the second cell's expression of each of a plurality of biomarkers. Each portion of the at least some images includes the second cell and a small region of interest around the second cell. FIG. 35 illustrates exemplary montages of two cells. Specifically, FIG. 35 illustrates a montage of a two cells, both in cluster 15 of a set of 20 clusters, where the left cell is taken from a normal field of view (GLO) whereas the right cell is from a Gleason grade 3 field of view (GL3). Some such embodiments of method 300, not covered by the claimed invention, further include displaying the montage of the first cell in the first cluster and the montage of the second cell in the first cluster simultaneously to enable a user to understand the feature of the first cluster. Similarly, montages of additional cells in the first cluster can be created and displayed.

FIG. 4 illustrates a method 400 of applying a modeled set of clusters to new cell profile data. The modeled set of clusters may be stored, for example, in storage device 116 or network device 126. The modeled set of clusters may be developed, for example, through method 200 taught herein.

Method 400 involves cell profile data relating to at least one field of view of at least one tissue sample from a patient. The cell profile data includes a multiplexed biometric image capturing the expression of a plurality of biomarkers. Individual cells in the field of view are delineated and segmenting into compartments. The resulting information is also included in the cell profile data. The method cell profile data may be stored, for example, in storage device 116 or network device 126.

Some embodiments of method 400, not covered by the claimed invention, further include obtaining the at least one tissue sample from the patient. Some embodiments of method 400, not covered by the claimed invention, further include staining and imaging the at least one tissue sample from the patient. Some embodiments of method 400, not covered by the claimed invention, further include delineating individual cells of the at least one tissue sample from the patient based on multiplexed images capturing the expression of each of the plurality of biomarkers. Some embodiments of method 400, not covered by the claimed invention, further include segmenting individual cells of the at least one tissue sample from the patient into compartments based on multiplexed images capturing the expression of each of the plurality of biomarkers.

In 420, the cells in the field of view of the at least one tissue sample are each assigned to a single cluster among a plurality of clusters of similar cells in a selected set of clusters. In some embodiments, not covered by the claimed invention, one or more processors, such as processors 102, 102', assign the cells to the appropriate clusters. Each cluster in the selected set of clusters comprises cells having a plurality of selected attributes more similar to the plurality of selected attributes of other cells in that cluster than to the plurality of selected attributes of cells in other clusters in the set. Cell similarity may be judged and clustering may done by any of the techniques discussed above with respect to 220. In some embodiments, not covered by the claimed invention, analysis code 150 includes the clustering algorithm. The set of clusters may have been selected by any of the techniques discussed above with respect to method 200.

In 430, a proportion of the cells assigned to each cluster in the selected set of clusters is observed. In some embodiments of method 400, not covered by the claimed invention, the observed proportion of cells is the observed proportion of the cells of each field of view assigned to each cluster. In some embodiments of method 400, not covered by the claimed invention, the observed proportion of cells is the observed proportion of the cells of each patient assigned to each cluster.

In 440, the observed proportions are examined for an association with a diagnosis, a prognosis, or a response to treatment of a condition or a disease. The association can be derived from a known association of the selected set of clusters with at least one piece of meta-information including a field of view level assessment or a patient-level assessment. The association may become known, for example, through analysis in accordance with method 200. In some embodiments, the association is an association with a Gleason tissue grade. In some embodiments, the association is an association with a disease or condition survival time.

Some embodiments of method 400, not covered by the claimed invention, further comprise examining the observed proportions in the selected set of clusters for a univariate association that can be derived from a known univariate association of the selected set of clusters. Other embodiments of method 400, not covered by the claimed invention, further comprise examining the observed proportions in the selected set of clusters for a multivariate association that can be derived from a known multivariate association of the selected set of clusters.

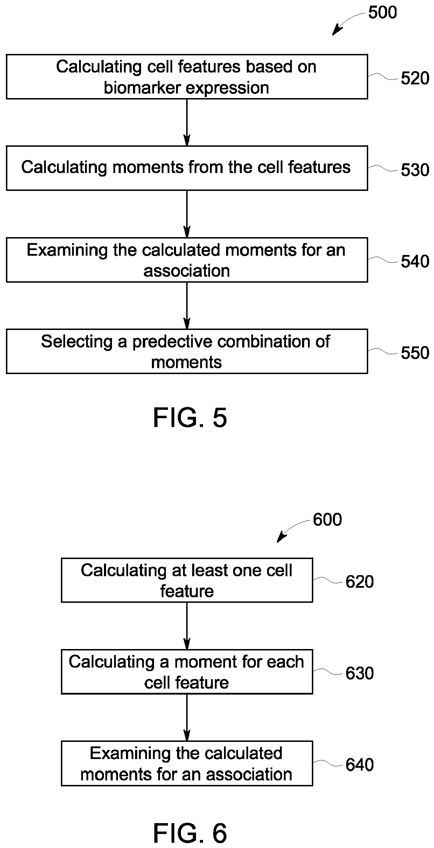

FIG. 5 illustrates a method 500 of developing a model for identifying a predictive set of moments of cell features from a data set in accordance with embodiments taught herein. The method leverages a data set that may be stored, for example, in storage device 116 or network device 126. The data set comprises cell profile data. The cell profile data includes multiplexed biometric images capturing the expression of a plurality of biomarkers with respect to a plurality of fields of view in which individual cells are delineated and segmenting into compartments. The cell profile data is generated from a plurality of tissue samples drawn from a cohort of patients having a commonality. The commonality may be, for example, that the patients share a disease or condition. Alternatively, the commonality may be, for example, that the patients share a preliminary diagnosis of the same disease or condition. The data set further comprises an association of the cell profile data with at least one piece of meta-information including a field of view level assessment or a patient-level assessment related to the commonality. The patient-level assessment may be, for example, survival time after surgery.

In 520, at least one cell feature is calculated based on the cell's expression of each of the plurality of biomarkers. Prior to calculating at least one cell feature, the cell profile data may be normalized. Some embodiments may normalize the measurement values to determine the mean and standard deviation of all the measurements associated with a given biomarker in a given study and subtract this mean value from each measurement value and then to divide the resultant difference by the standard deviation. In some embodiments, the measurement values are expressed on a log scale of the intensity of the expression of a biomarker in the image. A subtraction in measurement values expressed in the log scale in these embodiments may correspond to a division in the original raw measurement scale. Other embodiments may normalize the measurement values to determine the median intensity of a whole cell's expression for all cells within a batch of measurements and subtract this median value from each measurement value in the batch. Such median intensity may apply to the expression of a specific biomarker. This normalized or standardized value may be stored in the database or generated as part of the processing of the data set in the database.

Prior to calculating at least one cell feature, some embodiments filter a subset of the cell profile data from further calculations. Such filtering may be done as a quality control measure. Such filtering may exclude cell profile data related to cells comprising at least one compartment represented by fewer than a threshold number of pixels in the multiplexed image. Filtering may also be done for reasons beyond quality control. Such filtering may exclude the expression of each of the plurality of morphological biomarkers from further calculations. Accordingly, in some embodiments taught herein, calculating at least one cell feature involves calculating at least one cell feature based on the cell's expression of each of the plurality of non-morphological biomarkers.

Some embodiments of method 500 involve calculating two, three, four, or more cell features based on the cell's expression of each of the plurality of non-morphological biomarkers. In some embodiments, one or more processors, such as processors 102, 102', calculate the cell features. In some embodiments, analysis code 150 includes a definition for each cell feature. Cell features in some embodiments include a nucleus intensity ratio defined by subtracting half of the sum of the median intensity of the membrane and the median intensity of the cytoplasm from the median intensity of the cell nucleus's expression of at least one of the plurality of biomarkers. Cell features in some embodiments include a membrane intensity ratio defined by subtracting half of the sum of the median intensity of the nucleus and the median intensity of the cytoplasm from the median intensity of the cell membrane's expression of at least one of the plurality of biomarkers. Cell features in some embodiments include cytoplasm intensity ratio defined by subtracting half of the sum of the median intensity of the membrane and the median intensity of the nucleus from the median intensity of the cell cytoplasm's expression of at least one of the plurality of biomarkers.

In 530, a first moment is calculated for each of the plurality of fields of view from each of the cell features. In some embodiments, one or more processors, such as processors 102, 102', calculate the first moment of the cell feature. Embodiments taught herein may further involve calculating a second moment and/or a third moment for each of the plurality of fields of view from each of the cell features.

In 540, a plurality of combinations of attributes are examined for an association with the at least one piece of meta-information including the field of view level assessment or the patient-level assessment related to the commonality. The plurality of combinations of attributes at least include the calculated first moments. An association between the observed first moments of all biomarkers in a field of view and a field of view level assessment or a patient-level assessment can be derived by fitting a classification model with the assessment as the outcome and the biomarker first moments as the predictors. Several classification analysis frameworks exist, including random forests, neural networks, and logistic regression. For example, an association between tissue grade and the observed first moments of all biomarkers in a field of view is derived, in some embodiments, by fitting a random forest classification model with tissue grade as the outcome and the biomarker first moments as the predictors. An association between tissue grade and the observed first moments of all biomarkers in a field of view is derived, in other embodiments, by fitting a neural network classification model with tissue grade as the outcome and the biomarker first moments as the predictors. In some embodiments, the association is an association with the field of view level assessment of the sample, such a specific Gleason grade. In other embodiments, the association is an association with the patient-level assessment, such as a disease or condition survival time.

In some embodiments, one or more processors, such as processors 102, 102', examine the combinations. In embodiments that involve calculating a second moment, examining in 540 involves examining a plurality of combinations of attributes comprising the calculated first and second moments for an association with the at least one piece of meta-information including the field of view level assessment or the patient-level assessment related to the commonality. In embodiments that involve calculating a third moment, examining in 540 involves examining a plurality of combinations of attributes comprising the calculated first and third moments for an association with the at least one piece of meta-information including the field of view level assessment or the patient-level assessment related to the commonality. Some embodiments further involve examining the calculated first, second and third moments.

In some embodiments, the examining in 540 involves examining the calculated moments for a univariate association with the at least one piece of meta-information including the field of view level assessment or the patient-level assessment related to the commonality. In some embodiments, the examining in 540 involves examining the calculated moments for a multivariate association with the at least one piece of meta-information including the field of view level assessment or the patient-level assessment related to the commonality. In embodiment of method 500 in which second and/or third moments are calculated, the calculated moments can be examined for either a univariate or a multivariate association with the at least one piece of meta-information including the field of view level assessment or the patient-level assessment related to the commonality.

In some embodiments, the field of view level assessments are grouped. In cohorts of prostate cancer patients, for example, assessments resulting in a Gleason score of 2 or 3 may be grouped together. In these embodiments, the plurality of combinations of attributes are examined for an association with the grouped field of view level assessment related to the commonality of the patient cohorts. For example, combinations of attributes can be examined for an association with a low Gleason score where samples having a Gleason score of 2 or 3 are grouped together. Field of view level assessments of cohorts of other types of cancer may involve assessments of other types of tumors having their own relevant tumor grades. Other cancer grading systems include, for example, the Bloom-Richardson system for breast cancer and the Fuhrman system for kidney cancer. Whenever cancer or other diseases have assessments that may fall within more than two grades or categories, similar grades or categories may be grouped in some embodiments.

In 550, one of the plurality of combinations of attributes is selected based on a comparison of the performance of at least one model of the plurality of combinations of attributes. In some embodiments, visual display device 122 enables the selection to be made. Similar classification models can be created for each of the plurality of combinations of attributes. In some embodiments, one or more processors, such as processors 102, 102', create the classification models. Each model predicts an assessment based on the corresponding combination of attributes. In some embodiments, for example, each model predicts tissue grade based on a corresponding set of attributes. The performance of the model of each combination of attributes can be evaluated by various metrics of predictive performance in a test set of data not used for developing the model. Performance metrics that can be used to compare the combinations of attributes based on the models include sensitivity, specificity, and area under the receiver operating characteristic curve (also called concordance). The combination of attributes to be used may then be selected based on one or more of the model performance metrics. For example, in some embodiments, the combination of attributes associated with the highest concordance is selected. In other embodiments, the combination of attributes associated with the highest concordance is not selected due to apparent over-fitting of the data. For example, some embodiments involve selecting a combination of attributes based on a performance of the at least one model of the combination of attributes corresponding to a performance metric greater than a pre-defined threshold, which may be for example a concordance of 0.85 or greater. Other embodiments may involve selecting a combination based on the performance of a model of that combination in comparison with performance of models of other combinations. The selected combination of attributes comprises a predictive combination of attributes. Embodiments of method 500 may further include identifying at least one predictive non-morphological marker from the moments model.

FIG. 6 illustrates a method 600 of applying a model set of moments to new cell profile data in accordance with embodiments taught herein. The model set of moments may be stored, for example, in storage device 116 or network device 126. The model set of moments may be developed, for example, through any embodiments of method 500 taught herein.

Method 600 involves cell profile data relating to at least one field of view of at least one tissue sample from a patient. The cell profile data includes a multiplexed biometric image capturing the expression of a plurality of biomarkers. Individual cells in the field of view are delineated and segmenting into compartments. The resulting information is also included in the cell profile data. The cell profile data may be stored, for example, in storage device 116 or network device 126.

Some embodiments of method 600 further include obtaining the at least one tissue sample from the patient. Some embodiments of method 600 further include staining and imaging the at least one tissue sample from the patient. Some embodiments of method 600 further include delineating individual cells of the at least one tissue sample from the patient based on multiplexed images capturing the expression of each of the plurality of biomarkers. Some embodiments of method 600 further include segmenting individual cells of the at least one tissue sample from the patient into compartments based on multiplexed images capturing the expression of each of the plurality of biomarkers.

In 620, at least one cell feature is calculated based on the cell's expression of each of the plurality of biomarkers. In some embodiments, one or more processors, such as processors 102, 102', calculate at least one cell feature. In some embodiments, analysis code 150 includes a definition for each cell feature. The cell feature may be any cell feature discussed with respect to method 500. Some embodiments of method 600 further include calculating a plurality of cell features, which may include any combination of cell features discussed with respect to method 500. The cell features may be calculated from the cell's expression of non-morphological biomarkers.

In 630, a first moment is calculated for each cell feature for each of field of view. In some embodiments, one or more processors, such as processors 102, 102', calculate the first moment of the cell feature. Like method 500, method 600 may further include calculating a second and/or third moment for each cell feature.

In 640, the calculated first moments is examined for an association with a diagnosis, a prognosis, or a response to treatment of a condition or a disease. The association may be known from the model set of moments based on the existing data set, for example, such as described with respect to method 500. In some embodiments, the association is an association with a cell grade, such a specific Gleason grade. In other embodiments, the association is an association with a disease or condition survival time.

In embodiments of method 600 that involve calculating a second moment, examining in 640 involves examining the calculated first and second moments for an association with a diagnosis, a prognosis, or a response to treatment of a condition or a disease. In embodiments that involve calculating a third moment, examining in 640 involves examining the calculated first and third moments for an association with a diagnosis, a prognosis, or a response to treatment of a condition or a disease. Some embodiments further involve examining the calculated first, second and third moments.

In some embodiments, one or more processors, such as processors 102, 102', examine the calculated first moments. In some embodiments of method 600, examining in 640 involves examining the calculated first moments for a univariate association with a diagnosis, a prognosis, or a response to treatment of a condition or a disease. In other embodiments of method 600, examining in 640 involves examining the calculated first moments for a multivariate association with a diagnosis, a prognosis, or a response to treatment of a condition or a disease. In embodiment of method 600 in which second and/or third moments are calculated, the calculated moments can be examined for either a univariate or a multivariate association with a diagnosis, a prognosis, or a response to treatment of a condition or a disease.

**Exemplary Analysis and Visualization**

THE DATA SET

Analysis in accordance with exemplary methods taught herein was performed using information derived from tissue samples from a cohort of patients who had prostate surgery for cancer. Tissue samples may be defined as tissue cultures and include in vivo samples. Prostate tissue samples from 80 people were available for analysis. Of the contributing population, 62 had prostate cancer. Of those 62 prostate cancer patients, 11 were still alive at follow-up, 22 had died of the disease, and the remaining 29 had died of other causes. Table 1 gives population statistics for the contributing population on age, survival time and pathologist derived Gleason score for our data.

Table 1: Study Population Statistics

|           | All(n=80)     | CaP (n=62)  | Died of CaP (n=22) |
|-----------|---------------|-------------|--------------------|
| Age       | 70.9(10.2)    | 72.1(10.1)  | 76.2(11.9)         |
| SurvTime  | 8.76(6.49)    | 7.64(6.35)  | 3.73(3.44)         |
| Gleason 0 | 26(32%)       | 10(16%)     | 1(5%)              |
| 2-4       | 4(5%)         | 4(6%)       | 0                  |
| 5-6       | 13(16%) (?)   | 12(19%)     | 1(5%)              |
| 7         | 10(12%)       | 10(16%)     | 4(18%)             |
| 8-10      | 20(25%)       | 20(32%)     | 13(59%)            |
| Excluded  | 7(9%)         | 6(10%)      | 3(14%)             |

Other embodiments of the invention involve tissue samples from a cohort of patients sharing a different commonality. For example, one embodiment may involve tissue samples taken from a cohort of patients to determine if they had another form of cancer, such as breast cancer. Another embodiment may involve tissue samples taken from a cohort of patients to determine if they had another disease, such as Parkinson's disease. Similarly, other embodiments of the invention involve larger or smaller cohorts of patients.

The tissue samples were processed using fluorescence-based multiplexed immunohistochemistry. Fourteen biomarkers were used in the analysis. Five of the 14 biomarkers were used for segmentation and compartmentalization of individual cells: NaKATPase, PCAD, DAPI, S6, and Keratin. The remaining markers were AR, pmTOR, PI3Kp110a, PI3Kp85a, BetaCatenin, EGFR, CleavedCaspase3, pGSK3a, and CleavedPARP. All of the biomarkers passed a qualitative staining quality checks.

Other embodiments of the invention involve different biomarkers. Similarly, other embodiments of the invention involve more or fewer biomarkers.

After autofluorescence removal, illumination correction, and cell segmentation, the data included the median intensity for each protein image in the three compartments of each segmented cell in each field of view in all subjects. Cells were quality controlled by applying the following filters:

1. Cell does not overlap the background (edge areas of the image with incomplete marker data due to misregistration)
2. Cell has 2 or fewer segmented nuclei
3. Cell nucleus contains at least 50 pixels
4. Cell cytoplasm contains at least 50 pixels
5. Cell membrane contains at least 50 pixels

Other embodiments of the invention involve different quality control features. Similarly, other embodiments of the invention involve more or fewer quality control features.

After imaging, segmentation, and quality control, 54 patient subjects remained. The number of fields of view per patient ranged from 6 to 90. Of a total of 1757 fields of view imaged in the 54 subjects, 1349 fields of view contained sufficient tissue for analysis. Each of those 1349 fields of view were successfully graded by the team pathologist (QL).

In particular, Gleason scores were manually recorded for all fields of view by the team pathologist (QL) on a scale from 0 to 5. Due to scarcity of Gleason grade 2 data, the grade 2 fields of view were combined with Gleason grade 3 fields of view. Table 2 gives summaries of the fields of view-level Gleason grades.

Table 2: FOV-level Gleason Grades

| Died of Cancer | No | | | | Yes | | | |
|---|---|---|---|---|---|---|---|---|
| Age (years) | 48-72 | | 73-94 | | 48-72 | | 73-94 | |
| Survival Time (years) | 0-6 | 7-21 | 0-6 | 7-21 | 0-6 | 7-21 | 0-6 | 7-21 |
| Spot Gleason Grade | | | | | | | | |
| 0 | 64 | 304 | 99 | 29 | 7 | 18 | 63 | 36 |
| 2-3 | 32 | 54 | 36 | 10 | 9 | 3 | 13 | 9 |
| 4 | 34 | 73 | 24 | 1 | 8 | 11 | 125 | 38 |
| 5 | 11 | 3 | 3 | 0 | 0 | 6 | 120 | 20 |

Other embodiments of the invention may involve different field of view level assessments, which may be appropriate to the disease or condition affecting the relevant cohort of patients.

Subject samples were received and analyzed in 5 batches. Table 3 gives the Gleason score breakdown relative to the five batches, where entries are counts of tissue samples. Due to some subjects being analyzed in multiple batches, Table 3 includes 63 total tissue samples from the 54 unique subjects. Nine subjects had multiple tissue samples: 4 of these subjects were run in 2 batches, 2 were run in 3 batches, and 2 were run twice in a single batch. The last subject was run in 4 different batches.

Table 3: Subject-level Gleason scores in the 5 batches.

| Gleason Score | Batch 1 | Batch 2 | Batch 3 | Batch 4 | Batch 5 | Total |
|---|---|---|---|---|---|---|
| 0 | 1 | 0 | 1 | 4 | 4 | 10 |
| 2-4 | 3 | 0 | 0 | 0 | 1 | 4 |
| 5-6 | 4 | 4 | 3 | 2 | 1 | 14 |
| 7 | 3 | 1 | 3 | 2 | 0 | 9 |
| 8-10 | 4 | 7 | 9 | 4 | 2 | 26 |
| Total | 15 | 12 | 16 | 12 | 8 | 63 |

Disease-free survival was defined as time between surgery and death or follow-up. This measure was treated as right-censored if either the subject was alive at follow-up or died of a cause other than prostate cancer. Eighteen of the patient subjects died of prostate cancer before follow-up. The available post-surgery survival time for each patient subjects was also added to the data set thereby completing the raw data set.

Other embodiments of the invention may involve different patient level assessments, which may be appropriate to the disease or condition affecting the relevant cohort of patients.

Whole cell and compartment median intensities were normalized within each batch by subtracting the median of all whole-cell measurements for all cells in all subjects in the batch. For the 8 subjects who were analyzed in multiple batches, fields of view were batch-normalized, and then subsequently treated the same as subjects analyzed in a single batch. Other embodiments of the invention may involve more normalization, less normalization, different normalization, or no normalization of the data collected.

ADDITIONAL CELL FEATURES

Independently for each protein, four cell features were calculated from the cell level data. The four features, each defined on a log2 scale, were the median intensity of the whole cell, a nucleus intensity ratio, a membrane intensity ratio, and a cytoplasm intensity ratio. The three compartment ratios relate the median intensity of the expression of the nucleus, membrane, or cytoplasm to the average median intensity of the other two compartments. The three compartment ratios were defined as follows:

$$R_n = I_n - \left(I_m + I_c\right)/2$$

$$R_m = I_m - \left(I_n + I_c\right)/2$$

$$R_c = I_c - \left(I_m + I_n\right)/2$$

wherein $I_n$, $I_m$, and $I_c$ are the median intensity on a log2 scale in the nucleus, membrane, and cytoplasm, respectively. The compartment marker expression levels, e.g. membrane NaKATPase, were interpreted as the ratio of one compartment to the average of the other two as described. Other embodiments of the invention may involve more, less, or different cell features.

The data set described above was stored. Any additional cell features that are calculated may be added to the stored data set.

THE CLASSIFICATION AND SURVIVAL MODELS

Two distinct types of analysis-moments and cell cluster analysis-were conducted. The results of each type of analysis was then independently compared to classification and survival models.

For the field of view level assessment models, embodiments of the invention applied a Random Forest classifier, such as described in L. Breiman's "Random Forests" in Machine Learning 45(1), 5-32 (2001), with features described above. The outcome was two separate models related to the field of view Gleason grades. The first model distinguished Gleason grades (i.e., 2, 3, 4, or 5) fields of view from fields of view with Gleason grade 0. The second model distinguished Gleason grades 4 or 5 fields of view from Gleason grades 2 or 3 fields of view. In second model, fields of view with Gleason grade 0 were removed from analysis. The random Forest package (v. 4.5-36) for R (v. 2.11.0) was used with default settings. Out-of-bag error rates converged after 200 trees were constructed, so 500 trees were used for the classifier. During fitting, data was sampled and stratified by subject (using the strata argument to random Forest) to avoid overweighting subjects with an abundance of fields of view. Receiver Operating Characteristic (ROC) analysis were conducted by thresholding the predicted class probabilities from the out-of-bag predictions. The area under the ROC curve (AUC) was estimated. Variable importance results were based on decrease in classification accuracy when data from a given variable is scrambled. Variable dependence plots were based on predicted class log probabilities. Other embodiments of the invention may use more, less, or different field of view level assessment models.

For the association with survival (a patient-level outcome) an average was recorded of the spot-level features over the subject's invasive fields of view (Gleason score > 0) and a second average over the subject's normal fields of view. Subjects with no fields of view of a particular type had their marker feature data imputed by the population median.

For the patient level assessment models, embodiments of the invention applied a random survival forest model, such as disclosed in H. Ishwaran et al.'s "Random Survival Forests" in the Ann. App. Statist. 2:841-860 (2008). The random Survival Forest package (v. 3.6.3) for R (v. 2.11.0) was used with default arguments. Five thousand trees were used to build the model. The error metric tabulated was one minus Harrell's concordance index the probability that, in a randomly selected pair of subjects, the subject that dies first had a worse model-predicted outcome. According to Harrell, F.E. et al. in "Evaluating the Yield of Medical Tests," J. Amer. Med. Assoc. 247:2543-2546 (1982), 50% error is the random model, 0% is a perfect model. Other embodiments of the invention may use more, less, or different patient level assessment models.

This error metric was estimated on out-of-bag samples. Variable importance results were based on increase in concordance error for a given feature when random daughter assignments were used on tree nodes concerning a feature. Partial variable dependence plots were based on relative mortality, which is the predicted death rate in the population as a function of a given feature observed consistently in every subject in the population. Further, 3 separate binary classification models were fit to the survival data by setting a time threshold at 3, 5, and 10 years, and classifying whether the patient died of prostate cancer before the threshold.

MOMENTS ANALYSIS

In the moments-based analysis of embodiments of the invention, the four cell level features were summarized into field-of-view level statistics for association with the FOV-level Gleason grades. Based on the population of cells in the field of view, the mean, standard deviation, and skewness of all four expression-level features for all 14 markers were recorded. For association with the FOV grade, all 14 markers, including structural and target, were considered as predictors. This resulted in three moments for each of the four cell features for each of 14 biomarkers-for a total of168 FOV attributes. Other embodiments of the invention may involve more, less, or different field of view level attributes.

For example, the following cell morphological features from the single cell segmentation may be included in the moments-based models in various embodiments: Eccentricity_Cell, Solidity_Cell MajorAxisLength_Cell, MajorAxisAngle_Cell, Perimeter_Cell, Area_Cell, Area_Nuclei, Area_Mem, and Area_Cyto.

PREDICTING FIELD OF VIEW ASSESSMENTS USING THE MOMENTS ANALYSIS

During the field of view assessment model building, three options were considered with respect to the FOV attributes:

(1) whether to include the features based on the fluorescence data;

(2) whether to include the cell morphological data; and

(3) which order of moments of the fluorescence data to include: mean (m1); mean and standard deviation (m12); or mean, standard deviation, and skewness (m123).

Other embodiments of the invention may consider more, less, or different options with respect to the field of view attributes.

Table 4 gives the performance of the classifiers comparing cancerous (Gleason 2, 3, 4, or 5) versus normal grade (Gleason 0) fields of view based on different moments-based feature sets. Multiple combinations of FOV attributes were tried all including at least one of the order of moments (ml, m12, or m123). Some combinations included the fluorescence marker data, and some included the cell morphology features. The Area Under the ROC Curve (AUC) was at least 98% for all models that included at least the first moment of the fluorescent marker data. The morphological features increased the AUC only slightly.

Table 4: Performance of Moments based classifiers on Cancer vs. Normal Fields of View

| Moments Included | Fluorescence Features Included | Morphological Features Included | AUC |
|---|---|---|---|
| m12 | Yes | Yes | 0.983 |
| m1 | Yes | Yes | 0.982 |
| m123 | Yes | Yes | 0.982 |
| m123 | Yes | No | 0.982 |
| m12 | Yes | No | 0.981 |
| m1 | Yes | No | 0.980 |
| m12 | No | Yes | 0.896 |
| m123 | No | Yes | 0.892 |
| m1 | No | Yes | 0.845 |

Table 5 gives the performance of the classifiers comparing high grade (Gleason 4 or 5) versus low grade (Gleason 2 or 3) cancerous fields of view. Again, AUC suffered in models which did not include at least the first moment of the fluorescent marker data.

Table 5: Performance of Moments based classifiers on high grade vs. low grade Cancer Fields of View

| Moments Included | Fluorescence Features Included | Morphological Features Included | AUC |
|---|---|---|---|
| m12 | Yes | No | 0.929 |
| m12 | Yes | Yes | 0.928 |
| m1 | Yes | No | 0.928 |
| m123 | Yes | Yes | 0.928 |
| m1 | Yes | Yes | 0.926 |

(continued)

| Moments Included | Fluorescence Features Included | Morphological Features Included | AUC |
|---|---|---|---|
| m123 | Yes | No | 0.926 |
| m12 | No | Yes | 0.834 |
| m123 | No | Yes | 0.817 |
| m1 | No | Yes | 0.781 |

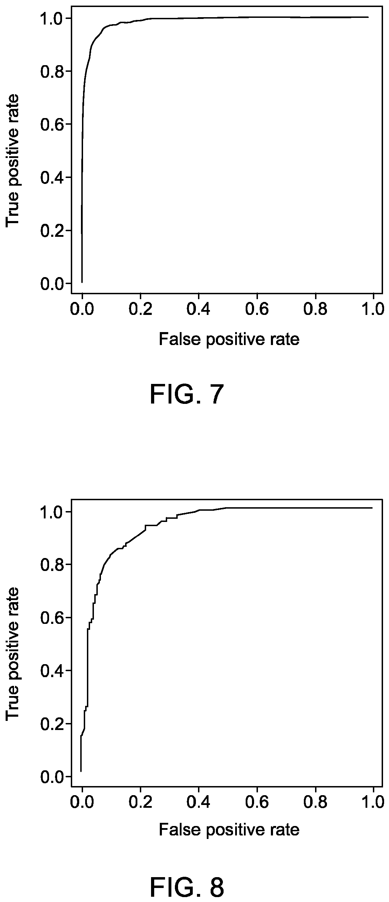

The ROC curves for the top models are given in FIGS. 7 and 8.

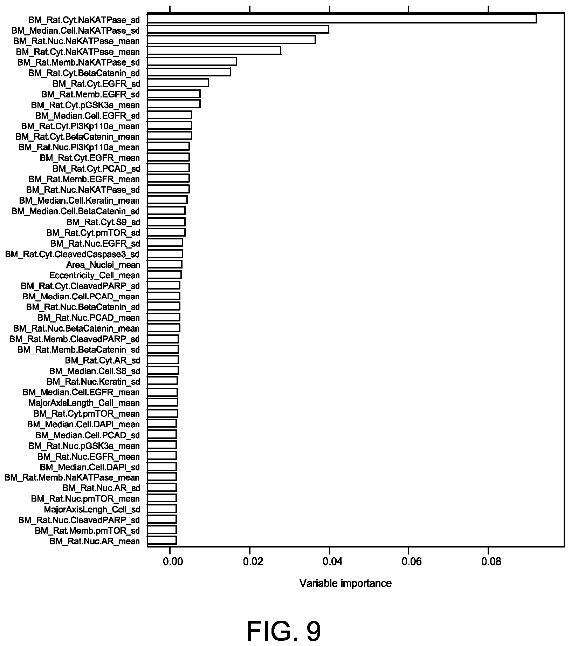

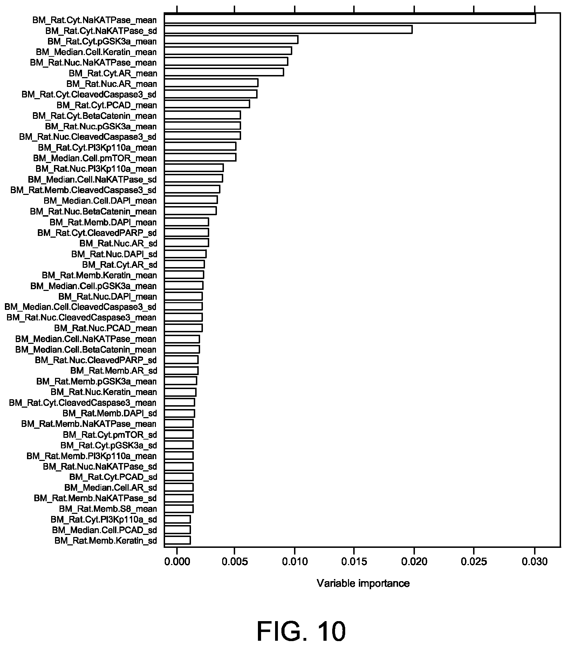

The variable importance plots for the top models are given in FIGS. 9 and 10. In both cases, the top features are related to NaKATPase, either being quantified outside the membrane or having high FOV-level standard deviation. The first morphological feature in the cancer/ normal classifier is area of the nucleus at 24th on the list.

PREDICTING PATIENT LEVEL ASSESSMENTS USING MOMENTS ANALYSIS

During patient level assessment model building, four options were considered with respect to the FOV attributes:

(1) whether to include the features based on the fluorescence data;

(2) whether to include the cell morphological data;

(3) which order of moments of the fluorescence data to include: mean (m1); mean and standard deviation (m12); or mean, standard deviation, and skewness (m123); and

(4) which fields of view from patient to include: invasive only, normal only, all, or the average in invasive tissues minus the average in normal tissues.

Other embodiments of the invention may consider more, less, or different options with respect to the field of view attributes.

Table 6 shows performance metrics for all the moments-based models fitted to the whole patient dataset. In the "FOVs included" column, the code "inv-norm" means that the feature used for the subject was the difference between the average seen in their invasive fields of view minus the average observed in their normal fields of view. In certain instances, the model with only age and Gleason score was fit 11 times and these rows are highlighted in bold. The different results for the 11 bold rows are related to random sampling error inherent to the random survival forest and random forest procedures.

The model with marker first moments in invasive fields of view and no morphological features was the preferred model. Although there are models which exceed it on RSF concordance metric, this model has better 3 year and 10 year AUC, and is only 0.8% less than the model which includes first and second moments. Further, this model increases the 5 year AUC over the null model from 73% to 93%. None of the models strongly exceed the null model's RSF concordance.

Table 7 gives the same performance metrics on models applied to the patient dataset excluding patients with Gleason scores greater than 0. The top model in Table 7, which includes first moment of marker features in invasive Fields of view, strongly improves on the null model in RSF concordance (69% ->81%), 5 year AUC (68% -> 89%), and 10 year AUC (64% -> 87%). As in Table 6, the rows of Table 7 highlighted in bold are those for which only age and Gleason score were included.

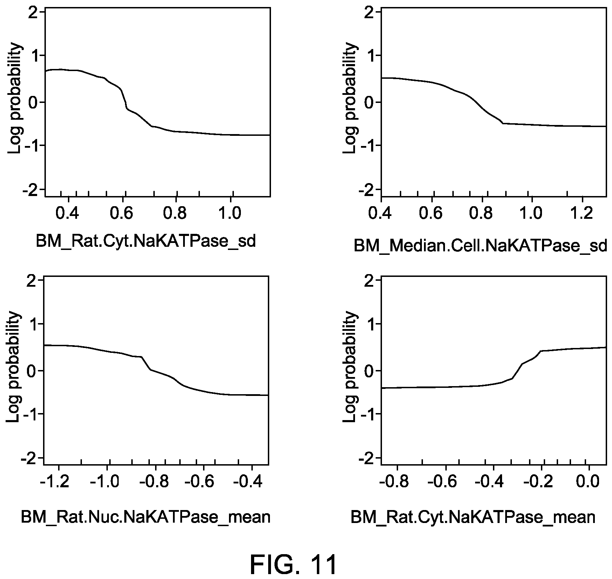

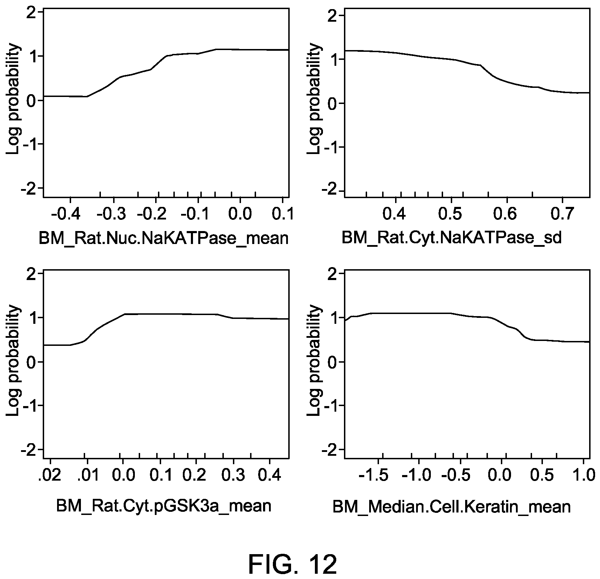

The partial dependence plots for the top 4 features in the two top models are given in FIGS. 11 and 12.

Table 6: Performance metrics on all moments-based models applied to the survival data including all subjects.

| Moments Included | Fluorescence Features Included | Morphological Features Included | FOVs Included | RSF Concordance | 3YR AUC | 5YR AUC | 10YR AUC |
|---|---|---|---|---|---|---|---|
| m12 | Yes | No | inv | 0.810 | 0.891 | 0.938 | 0.827 |
| m123 | Yes | No | inv+norm | 0.808 | 0.878 | 0.901 | 0.791 |
| m12 | Yes | No | inv+norm | 0.807 | 0.918 | 0.916 | 0.807 |

(continued)

| Moments Included | Fluorescence Features Included | Morphological Features Included | FOVs Included | RSF Concordance | 3YR AUC | 5YR AUC | 10YR AUC |
|---|---|---|---|---|---|---|---|
| m1 | Yes | No | inv+norm | 0.805 | 0.920 | 0.934 | 0.856 |
| m123 | Yes | Yes | inv | 0.804 | 0.904 | 0.901 | 0.843 |
| m1 | Yes | No | inv | 0.802 | 0.931 | 0.932 | 0.852 |
| m12 | Yes | Yes | inv | 0.801 | 0.876 | 0.914 | 0.830 |
| m12 | Yes | Yes | inv+norm | 0.799 | 0.887 | 0.901 | 0.807 |
| m1 | Yes | Yes | inv+norm | 0.799 | 0.900 | 0.934 | 0.836 |
| m123 | Yes | No | inv | 0.799 | 0.889 | 0.870 | 0.813 |
| m123 | Yes | Yes | i nv+norm | 0.798 | 0.927 | 0.883 | 0.830 |
| m1 | Yes | Yes | inv | 0.793 | 0.898 | 0.927 | 0.856 |
| **m1** | **No** | **No** | **norm** | **0.776** | **0.893** | **0.744** | **0.706** |
| **m123** | **No** | **No** | **inv-norm** | **0.769** | **0.887** | **0.721** | **0.705** |
| **m1** | **No** | **No** | **inv-norm** | **0.769** | **0.871** | **0.741** | **0.711** |
| **m12** | **No** | **No** | **inv-norm** | **0.767** | **0.887** | **0.720** | **0.692** |
| m1 | No | Yes | inv-norm | 0.766 | 0.869 | 0.786 | 0.751 |
| **m123** | **No** | **No** | **inv+norm** | **0.764** | **0.891** | **0.737** | **0.703** |
| **m123** | **No** | **No** | **inv** | **0.764** | **0.878** | **0.749** | **0.714** |
| **m1** | **No** | **No** | **inv** | **0.764** | **0.867** | **0.734** | **0.703** |
| **m12** | **No** | **No** | **inv+norm** | **0.763** | **0.882** | **0.722** | **0.710** |
| **m123** | **No** | **No** | **norm** | **0.763** | **0.878** | **0.741** | **0.704** |
| **m1** | **No** | **No** | **inv+norm** | **0.760** | **0.869** | **0.751** | **0.699** |
| **m12** | **No** | **No** | **inv** | **0.760** | **0.878** | **0.744** | **0.690** |
| **m12** | **No** | **No** | **norm** | **0.755** | **0.880** | **0.729** | **0.707** |
| m1 | No | Yes | inv | 0.749 | 0.836 | 0.766 | 0.800 |
| m12 | No | Yes | inv-norm | 0.735 | 0.847 | 0.697 | 0.740 |
| m123 | No | Yes | inv-norm | 0.726 | 0.824 | 0.672 | 0.729 |
| m1 | No | Yes | norm | 0.726 | 0.856 | 0.652 | 0.675 |
| m1 | No | Yes | inv+norm | 0.715 | 0.811 | 0.760 | 0.759 |
| m123 | No | Yes | inv | 0.712 | 0.884 | 0.810 | 0.746 |
| m12 | No | Yes | inv | 0.712 | 0.833 | 0.755 | 0.772 |
| m12 | No | Yes | norm | 0.705 | 0.760 | 0.648 | 0.616 |
| m1 | Yes | No | norm | 0.703 | 0.848 | 0.791 | 0.781 |
| m123 | No | Yes | inv+norm | 0.700 | 0.847 | 0.782 | 0.751 |
| m12 | No | Yes | inv+norm | 0.693 | 0.824 | 0.701 | 0.721 |
| m1 | Yes | Yes | inv-norm | 0.686 | 0.887 | 0.793 | 0.731 |
| m1 | Yes | Yes | norm | 0.682 | 0.773 | 0.745 | 0.724 |
| m1 | Yes | No | inv-norm | 0.680 | 0.831 | 0.784 | 0.688 |

(continued)

| Moments Included | Fluorescence Features Included | Morphological Features Included | FOVs Included | RSF Concordance | 3YR AUC | 5YR AUC | 10YR AUC |
|---|---|---|---|---|---|---|---|
| m12 | Yes | Yes | inv-norm | 0.671 | 0.822 | 0.755 | 0.678 |
| m12 | Yes | No | norm | 0.670 | 0.698 | 0.777 | 0.656 |
| m123 | No | Yes | norm | 0.668 | 0.744 | 0.608 | 0.559 |
| m12 | Yes | No | inv-norm | 0.653 | 0.840 | 0.824 | 0.655 |
| m123 | Yes | Yes | inv-norm | 0.651 | 0.829 | 0.663 | 0.649 |
| m123 | Yes | No | norm | 0.651 | 0.744 | 0.701 | 0.631 |
| m12 | Yes | Yes | norm | 0.627 | 0.638 | 0.672 | 0.622 |
| m123 | Yes | No | inv-norm | 0.624 | 0.804 | 0.767 | 0.616 |
| m123 | Yes | Yes | norm | 0.607 | 0.691 | 0.685 | 0.639 |

Table 7: Performance metrics on all moments-based models applied to the survival data including subjects with Gleason score > 0.

| Moments Included | Fluorescence Features Included | Morphological Features Included | FOVs Included | RSF Concordance | 3YR AUC | 5YR AUC | 10YR AUC |
|---|---|---|---|---|---|---|---|
| m1 | Yes | No | inv | 0.812 | 0.869 | 0.892 | 0.875 |
| m12 | Yes | No | inv+norm | 0.802 | 0.883 | 0.901 | 0.843 |
| m12 | Yes | Yes | inv+norm | 0.800 | 0.831 | 0.870 | 0.790 |
| m1 | Yes | No | inv+norm | 0.800 | 0.886 | 0.897 | 0.875 |
| m1 | Yes | Yes | inv | 0.798 | 0.860 | 0.889 | 0.879 |
| m123 | Yes | No | inv+norm | 0.792 | 0.817 | 0.875 | 0.810 |
| m12 | Yes | No | inv | 0.792 | 0.851 | 0.885 | 0.860 |
| m12 | Yes | Yes | inv | 0.790 | 0.834 | 0.880 | 0.834 |
| m1 | Yes | Yes | inv+norm | 0.788 | 0.880 | 0.897 | 0.869 |
| m123 | Yes | Yes | inv+norm | 0.786 | 0.831 | 0.839 | 0.851 |
| m123 | Yes | No | inv | 0.786 | 0.840 | 0.875 | 0.825 |
| m123 | Yes | Yes | inv | 0.781 | 0.897 | 0.892 | 0.851 |
| m1 | No | Yes | inv-norm | 0.767 | 0.886 | 0.702 | 0.782 |
| m12 | No | Yes | inv-norm | 0.742 | 0.817 | 0.647 | 0.735 |
| m1 | No | Yes | inv | 0.720 | 0.800 | 0.736 | 0.790 |
| m123 | No | Yes | inv-norm | 0.713 | 0.814 | 0.615 | 0.724 |
| **m123** | **No** | **No** | **inv-norm** | **0.705** | **0.849** | **0.692** | **0.662** |
| **m1** | **No** | **No** | **norm** | **0.703** | **0.851** | **0.690** | **0.647** |
| **m12** | **No** | **No** | **inv-norm** | **0.701** | **0.857** | **0.656** | **0.644** |
| **m1** | **No** | **No** | **inv-norm** | **0.697** | **0.854** | **0.695** | **0.649** |
| **m123** | **No** | **No** | **inv+norm** | **0.697** | **0.849** | **0.695** | **0.640** |
| **ml2** | **No** | **No** | **inv+norm** | **0.695** | **0.834** | **0.675** | **0.640** |

(continued)

| Moments Included | Fluorescence Features Included | Morphological Features Included | FOVs Included | RSF Concordance | 3YR AUC | 5YR AUC | 10YR AUC |
|---|---|---|---|---|---|---|---|
| **m1** | **No** | **No** | **inv** | **0.695** | **0.837** | **0.675** | **0.644** |
| **m123** | **No** | **No** | **norm** | **0.693** | **0.849** | **0.675** | **0.634** |
| m1 | No | Yes | inv+norm | 0.691 | 0.749 | 0.620 | 0.763 |
| **m12** | **No** | **No** | **inv** | **0.691** | **0.857** | **0.678** | **0.642** |
| **m12** | **No** | **No** | **norm** | **0.689** | **0.840** | **0.675** | **0.651** |
| **m1** | **No** | **No** | **inv+norm** | **0.687** | **0.843** | **0.673** | **0.640** |
| **m123** | **No** | **No** | **inv** | **0.678** | **0.849** | **0.691** | **0.627** |
| m123 | No | Yes | inv | 0.676 | 0.847 | 0.764 | 0.763 |
| m12 | No | Yes | inv | 0.676 | 0.771 | 0.690 | 0.738 |
| m1 | Yes | Yes | inv-norm | 0.654 | 0.866 | 0.728 | 0.696 |
| m123 | No | Yes | inv+norm | 0.650 | 0.786 | 0.757 | 0.744 |
| m12 | No | Yes | inv+norm | 0.649 | 0.760 | 0.584 | 0.642 |
| m12 | Yes | Yes | inv-norm | 0.639 | 0.840 | 0.695 | 0.670 |
| m1 | No | Yes | norm | 0.629 | 0.740 | 0.563 | 0.653 |
| m123 | Yes | Yes | inv-norm | 0.621 | 0.800 | 0.650 | 0.610 |
| m1 | Yes | No | inv-norm | 0.617 | 0.853 | 0.716 | 0.664 |
| m123 | No | Yes | norm | 0.610 | 0.637 | 0.464 | 0.509 |
| m12 | No | Yes | norm | 0.606 | 0.709 | 0.486 | 0.614 |
| m12 | Yes | No | inv-norm | 0.594 | 0.820 | 0.728 | 0.677 |
| m1 | Yes | No | norm | 0.588 | 0.740 | 0.739 | 0.698 |
| m1 | Yes | Yes | norm | 0.579 | 0.691 | 0.690 | 0.657 |
| m123 | Yes | No | norm | 0.569 | 0.667 | 0.685 | 0.700 |
| m123 | Yes | No | inv-norm | 0.561 | 0.777 | 0.677 | 0.565 |
| m123 | Yes | Yes | norm | 0.548 | 0.649 | 0.647 | 0.631 |
| m12 | Yes | No | norm | 0.532 | 0.660 | 0.678 | 0.608 |
| m12 | Yes | Yes | norm | 0.518 | 0.617 | 0.611 | 0.584 |

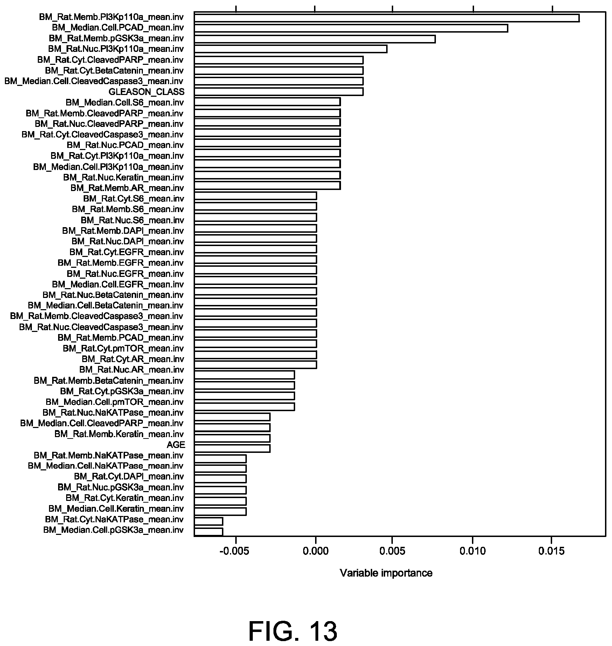

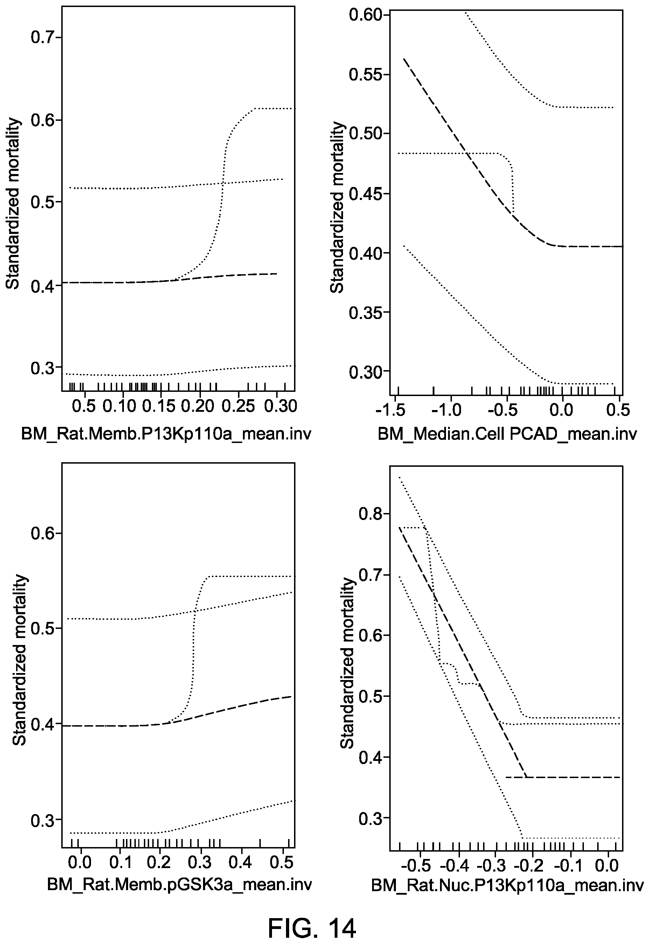

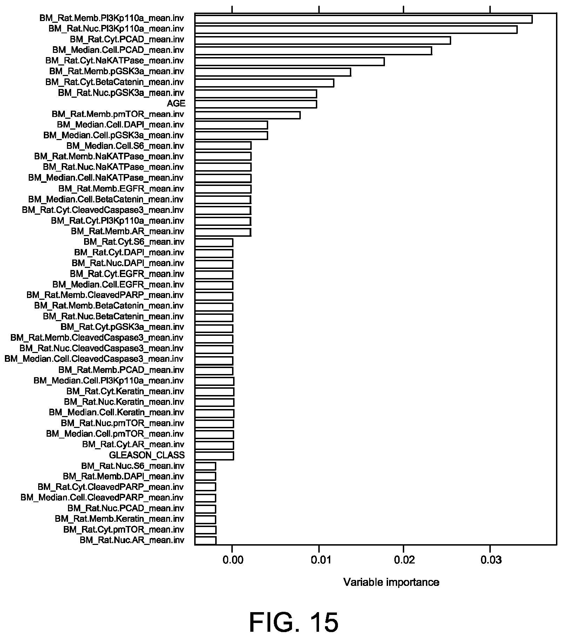

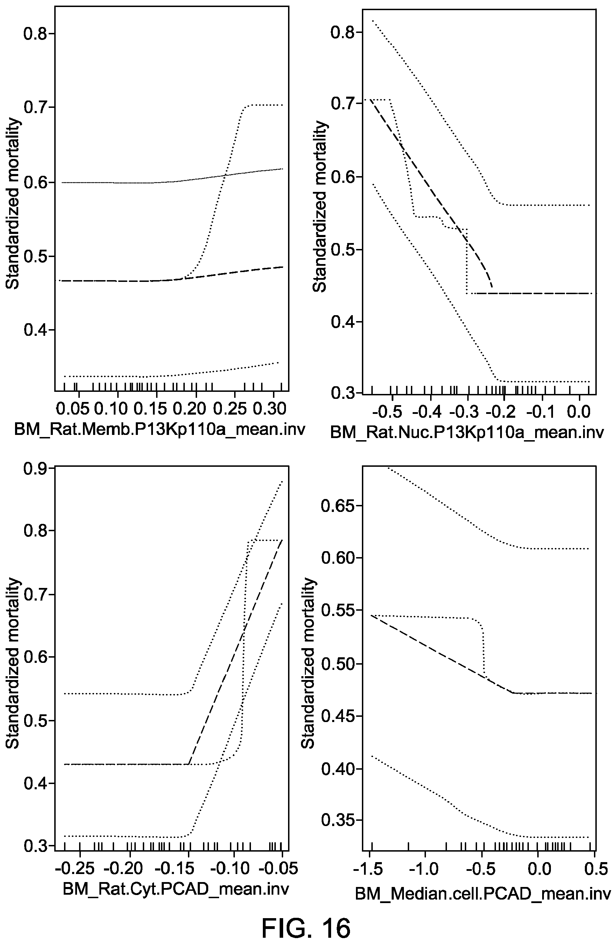

In the whole cohort analysis, PI3Kp110a, PCAD, and pGSK3a were the most predictive of the markers, as shown in FIG. 13. FIG. 14 shows that stronger membrane abundance of PI3Kp110 and pGSK3a, as well as low whole cell PCAD abundance, may be associated with shorter survival. In the cohort of subjects with Gleason score greater than 0, the list of important features was similar, as seen in FIGS. 15 and 16.

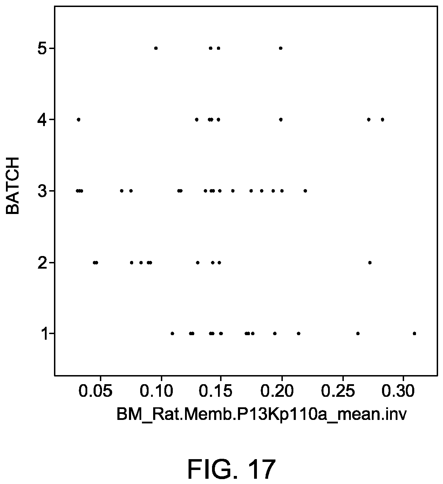

All top features were checked for obvious batch effects, none were found. See for example FIG. 17 where only a slight differential is seen in batch 1.

CELL CLUSTER ANALYSIS

In the cell clusters analysis of embodiments, not covered by the claimed invention, cells were clustered into K groups based on the 14 markers and the 4 cell-level features, a 56 dimensional marker space, using K-medians clustering on 20,000 cells sampled from the whole cohort stratified by subject. The stepFlexclust function of flexclust library (v. 1.3-1) for R (v. 2.11.0) was run with 20 replicates assuming K ranged between 2 and 50. Then every cell in the whole cohort was associated with one of the K clusters by computing distances from the cluster centroids. This was accomplished using the predict function in flexclust. FOV-level cell cluster features were then defined as the proportion of cells in the FOV belonging to each of the K clusters. Separate classification and survival models were fit for each of the sets of K groups generated. Other embodiments, not covered by the claimed invention, may use a different clustering algorithm, may apply the algorithm to a different set of cell attributes, may specify a different range of clusters sets to generate, or may identify specific numbers of clusters sets to generate.

PREDICTING FIELD OF VIEW LEVEL ASSESSMENTS USING CLUSTER ANALYSIS

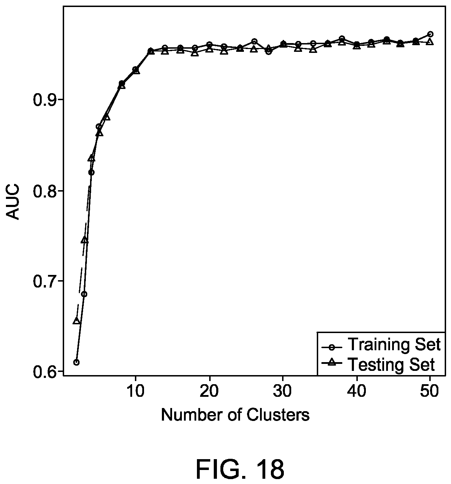

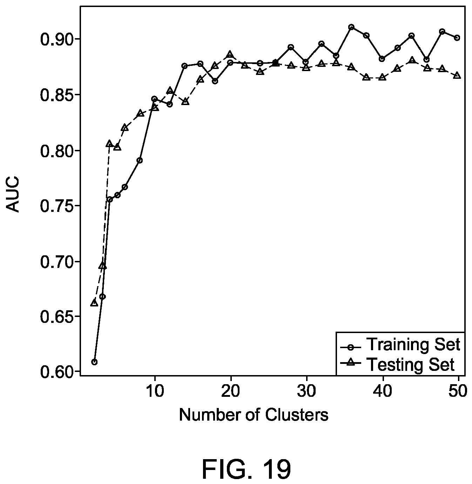

The performance of both the cancer versus normal field of view and the high grade versus low grade cancer field of view classifiers stabilized after including approximately 20 cell clusters, as seen in FIGS. 18 and 19. At 20 cell clusters, the normal versus cancer classifier AUCs were 96.1% and 95.7% in training and test sets, respectively. At 20 cell clusters, the high grade versus low grade cancer classifier AUCs were lower: 88.0% in training and 88.7% in test sets. Morphological features were not included in these models.

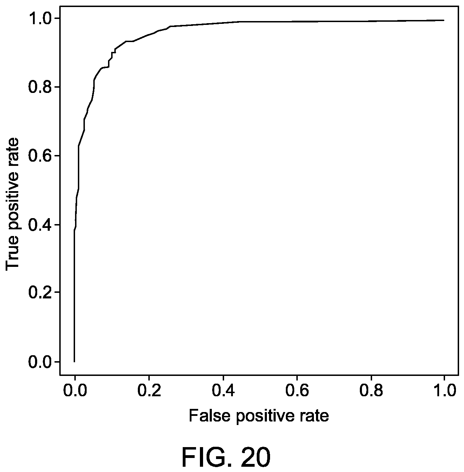

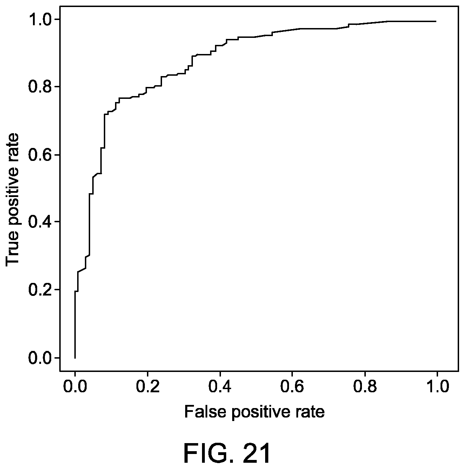

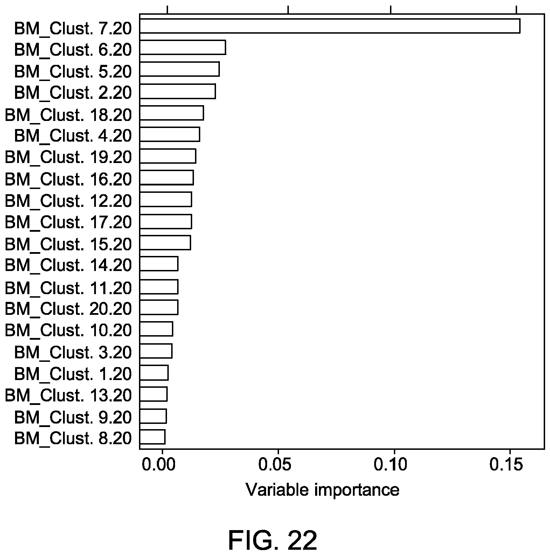

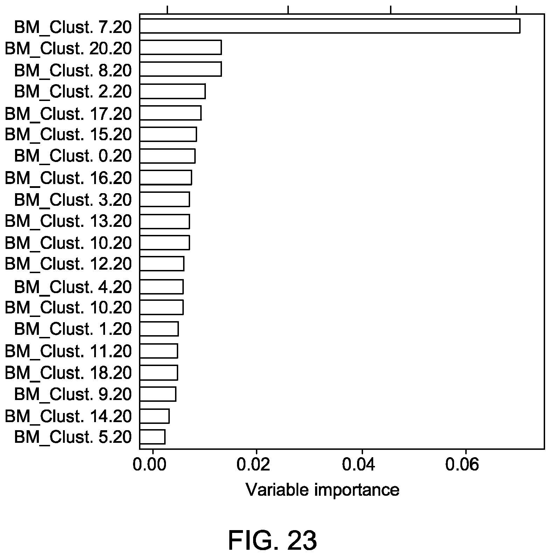

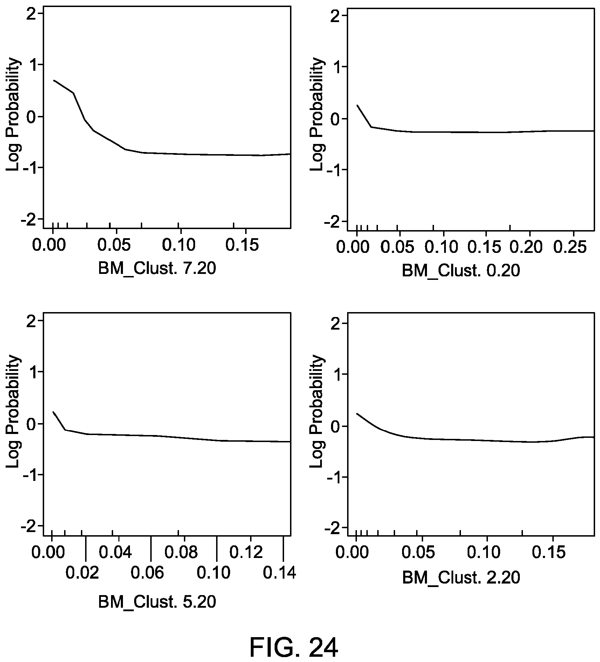

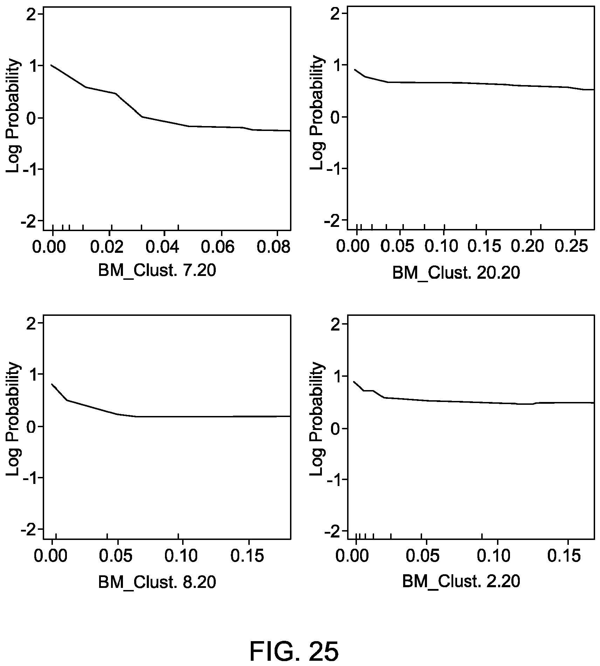

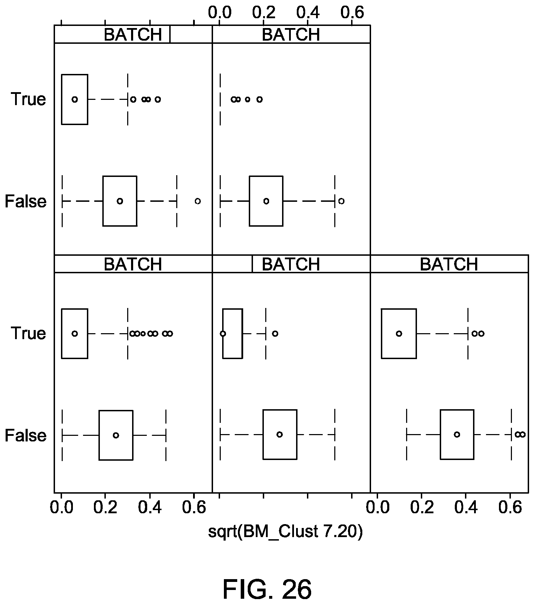

The ROC curves for the 20 cell cluster models are given in FIGS. 20 and 21. In both classifiers, cancer versus normal and high versus low grade cancer, the single cluster 7 stands out as being highly predictive of FOV grade, as shown in FIGS. 22 and 23. Cluster 7 is an indication of normal tissue as are the rest of the top 4 features in both models; see FIGS. 24 and 25. The pattern of lower abundance of cluster 7 cells in higher grade cancers was evident in all 5 batches, see FIG. 26.

The FOV proportions of cluster 7 cells were checked for batch effects and none were found.

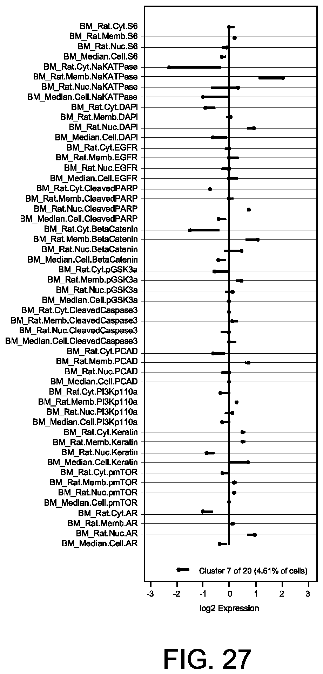

The signature of cluster 7 is plotted in FIG. 27. Significant features of this cluster are increased nuclear and membrane abundance of both NaKATPase and beta Catenin with associated decrease in cytoplasmic abundance of both.

PREDICTING PATIENT LEVEL ASSESSMENTS USING CLUSTER ANALYSIS

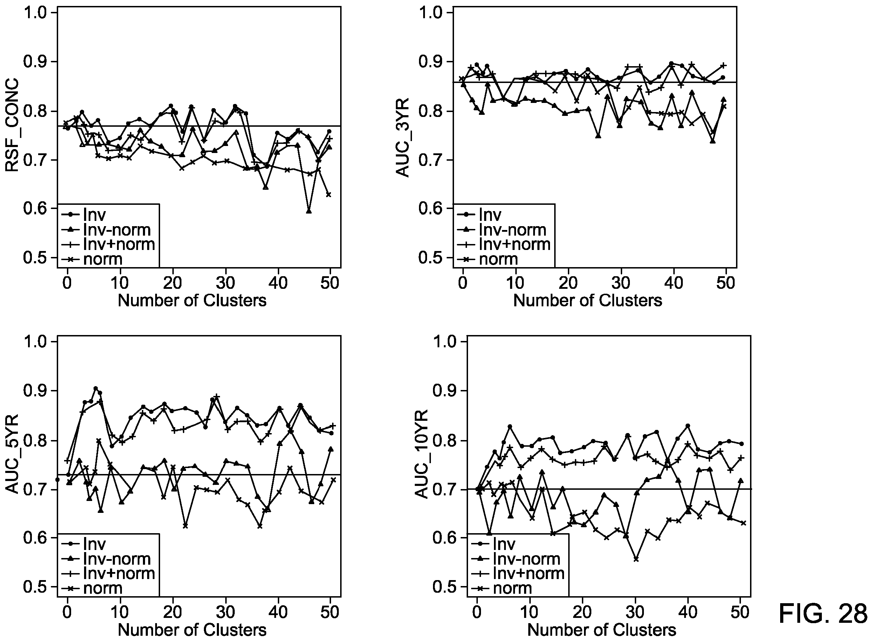

In the whole cohort analysis, only later time survival prediction can be improved somewhat over the null model with age and Gleason score. This is shown in FIG. 28 where the random survival forest concordance (RSF_CONC) and the AUC for classifying death of prostate cancer within 3, 5 and 10 years (AUC_*YR) are plotted vs. the number of clusters included in the model. Inclusion of invasive versus normal FOVs is differentiated by color in the figure. Models which may perform better than the null model are those which include invasive features, as these models showed improve survival predictions at 5 years and beyond. In general, 6 clusters will provide good performance.

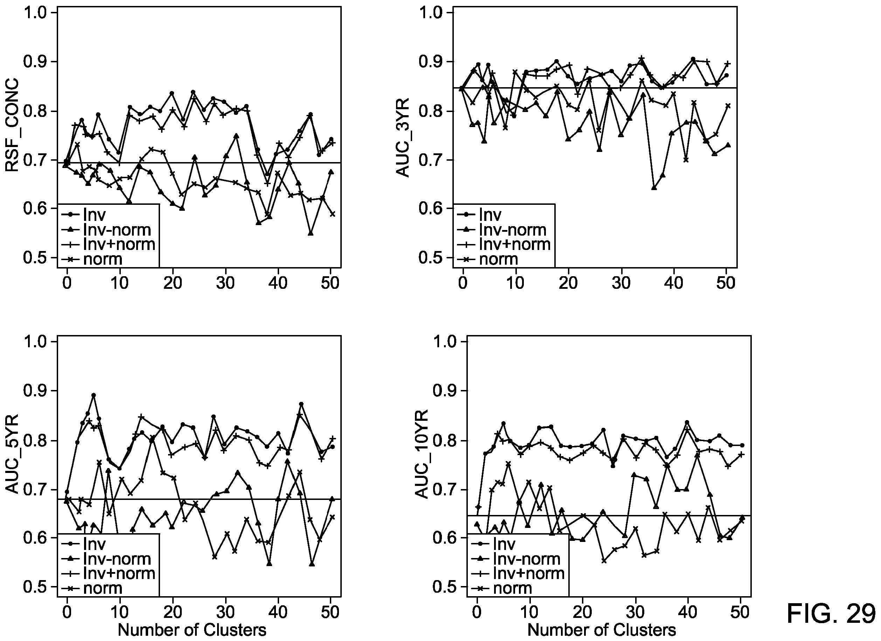

In the Gleason score greater than 0 cohort analysis, survival time concordance metric and 5- and 10-year death classification rates are better than the null model when including at least 5 cell clusters, see FIG. 29. Survival time concordance rises until approximately 20 clusters are included, whereas 5 year death is best classified with as few as 5 clusters. Including features from normal FOVs does not generally improve model performance.

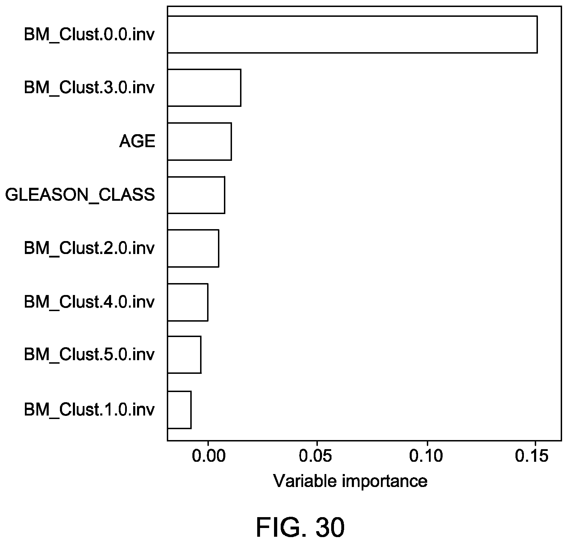

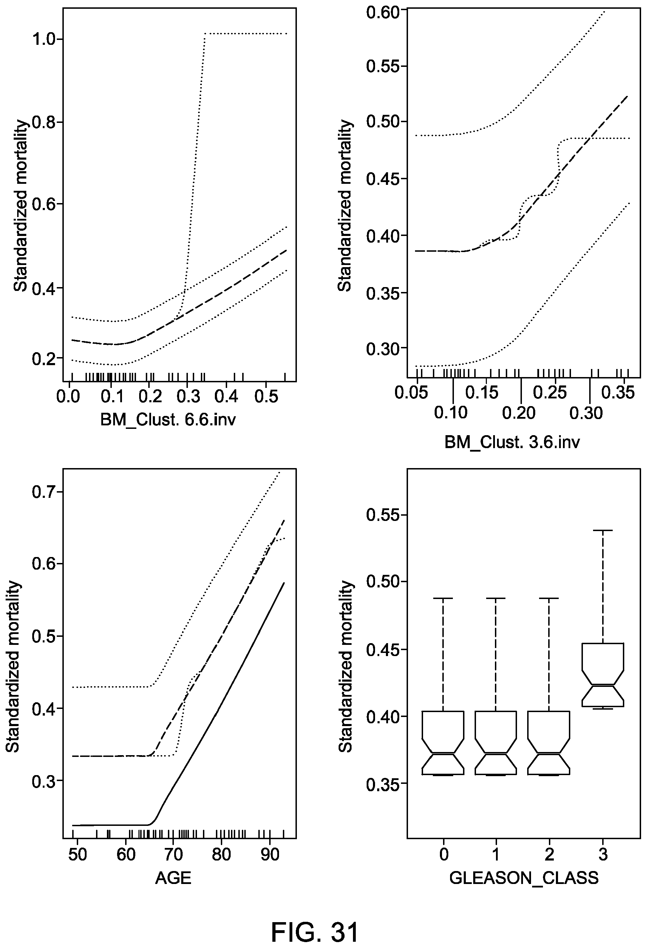

The variable importance plot for the model which included 6 clusters in invasive tissues applied to the whole cohort, in FIG. 30, shows that cluster 6 is much more predictive than any of the other 5 clusters in the model. Cluster 6 is associated with shorter survival time, as shown in FIG. 31.

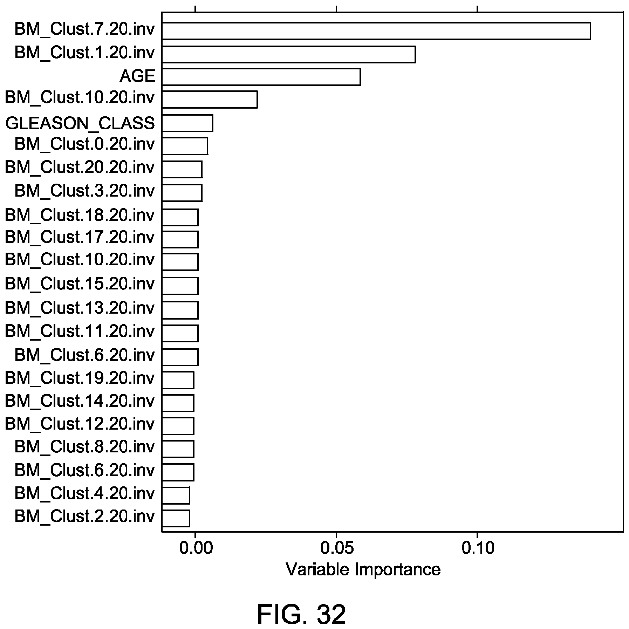

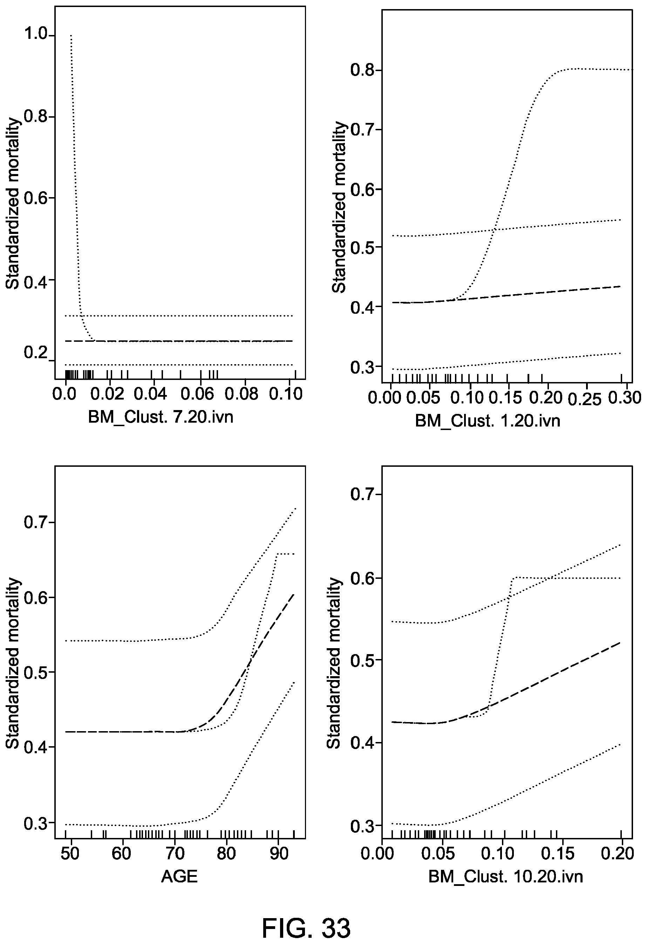

In the 20 cluster analysis of the Gleason score greater than 0 cohort, two clusters are relatively important in predicting survival time: 7 and 1. FIG. 32 is the variable importance of the survival model on the Gleason score greater than 0 cohort. Cluster 7 is associated with longer survival time, whereas cluster 1 is associated with shorter survival time. FIG. 33 is the partial dependence of the top four features in the 20 cluster model of the Gleason score greater than 0 cohort

All top clusters were checked for batch effects and none were found.

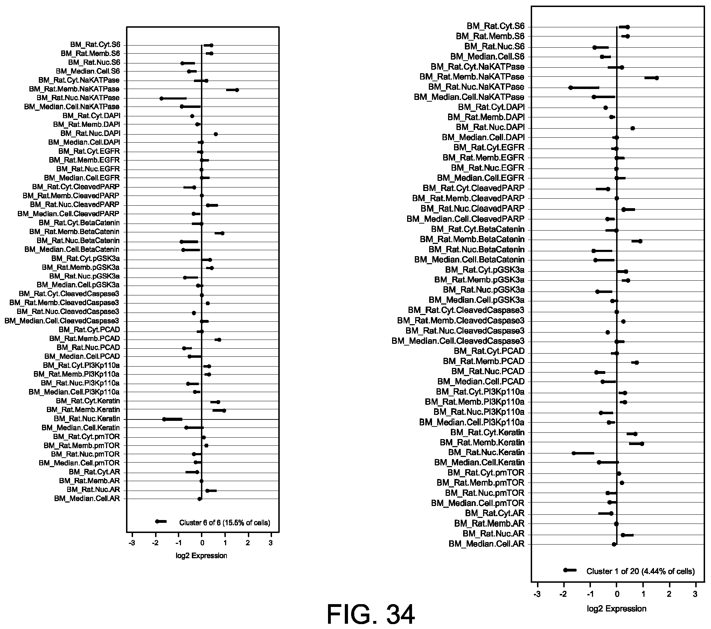

The signatures of clusters 6/6 and 1/20 are given in FIG. 34. These two clusters show similar signatures which are marked by accentuated localization in NaKATPase, S6, BetaCatenin, PCAD, PI3Kp110a, and Keratin. They also show somewhat low whole cell NaKATPase, BetaCatenin, and Keratin.

**Claims**

1.   A system for analyzing tissue features based on image data generated through multiplexing staining-destaining techniques, the system comprising:
     at least one processor for executing code that causes the at least one processor to perform the steps of:

          generating images of a plurality of stained fields of view, wherein the generated images illustrate the expression of a plurality of biomarkers within individual cells that are part of a larger cell sample, wherein the larger cell sample comprises at least one tissue sample from a patient,
          a storage device for storing cell profile data comprising the generated images capturing the expression of a plurality of biomarkers by the at least one tissue sample from a patient with respect to plurality of fields of view in which individual cells are delineated and segmented into compartments, and

the at least one processor for executing code that further causes the at least one processor to perform the steps of:

calculating at least one cell feature based on the cell's expression of each of the plurality of biomarkers;

calculating for each of the plurality of fields of view and for each of the at least one cell feature a first moment of the cell feature for the cells in the field of view, wherein the first moment is a selection of or mean, standard deviation, and skewness; and,

examining the calculated first moments for an association with a diagnosis, a prognosis, or a response to treatment of a condition or a disease, wherein the association is predicted from a predictive set of moments that has been identified from an existing data set, the data set comprises cell profile data generated from a plurality of tissue samples drawn from a cohort of patients having a commonality, the data set further comprises an association of the cell profile data with at least one piece of meta-information including a field of view level assessment or a patient-level assessment related to the commonality, the association describes an association between observed first moments and a field of view level assessment or a patient-level assessment which is derived by fitting a classification model with the assessment as an outcome and first moments as predictors, and the assessment is indicative of a diagnosis, a prognosis or a response to treatment of a condition or a disease.

2. The system of claim 1 further comprising a camera for digitally imaging the at least one tissue sample from the patient; optionally wherein the at least one processor further executes code that causes the at least one processor to perform the step of segmenting individual cells of the at least one tissue sample from the patient into compartments based on the generated images capturing the expression of each of the plurality of biomarkers.

3. The system of claim 1 or wherein the at least one processor further executes code that causes the at least one processor to perform the steps of:

calculating at least two cell features based on the cell's expression of each of the plurality of non-morphological biomarkers; and

calculating a first moment for each of the at least two cell features for each of the at least one field of view.

4. The system of claim 1 wherein the at least one processor further executes code that causes the at least one processor to perform the steps of:

calculating at least three cell features based on the cell's expression of each of the plurality of non-morphological biomarkers; and

calculating a first moment for each of the at least three cell features for each of the plurality of fields of view.

5. The system of claim 1 wherein the at least one processor further executes code that causes the at least one processor to perform the steps of:

calculating a second moment for each of the plurality of fields of view from each of the at least one cell feature; and examining the calculated first and second moments for an association with a diagnosis, a prognosis, or a response to treatment of a condition or a disease.

6. The system of claim 5 wherein the at least one processor further executes code that causes the at least one processor to perform the steps of:

calculating a third moment for each of the plurality of fields of view from each of the at least one cell feature; and examining the calculated first, second, and third moments for an association with a diagnosis, a prognosis, or a response to treatment of a condition or a disease.

7. The system of claim 6 wherein the moments comprise mean, standard deviation and skewness.

8. The system of claim 1 wherein the association with the diagnosis or the prognosis of the disease comprises a cell grade or a survival time.

**Patentansprüche**

1. System zum Analysieren von Gewebemerkmalen auf Basis von Bilddaten, welche durch Multiplexen von Anfärbe-Entfärbetechniken erzeugt werden, wobei das System umfasst:

   zumindest einen Prozessor zum Ausführen von Code, welcher den zumindest einen Prozessor veranlasst, die folgenden Schritte auszuführen:

   Erzeugen von Bildern einer Vielzahl von angefärbten Sichtfeldern, wobei die erzeugten Bilder den Ausdruck einer Vielzahl von Biomarkern innerhalb individueller Zellen darstellen, welche Teil einer größeren Zellprobe sind, wobei die größere Zellprobe zumindest eine Gewebeprobe von einem Patienten umfasst, eine Speichervorrichtung zum Speichern von Zellprofildaten, welche die erzeugten Bilder umfassen, welche den Ausdruck einer Vielzahl von Biomarkern durch die zumindest eine Gewebeprobe von einem Patienten in Bezug auf Vielzahl von Sichtfeldern erfassen, in welchen individuelle Zellprofildaten abgegrenzt und in Kammern segmentiert werden, und den zumindest einen Prozessor zum Ausführen von Code, welcher den zumindest einen Prozessor weiter veranlasst, die folgenden Schritte auszuführen:

   Berechnen zumindest eines Zellmerkmals auf Basis des Ausdrucks der Zelle jedes der Vielzahl von Biomarkern;
   Berechnen, für jedes der Vielzahl von Sichtfeldern und für jedes des zumindest einen Zellmerkmals, eines ersten Moments des Zellmerkmals für die Zellen in dem Sichtfeld, wobei das erste Moment eine Auswahl von oder Mittel, Standardabweichung und Schiefe ist; und
   Untersuchen der berechneten ersten Momente für eine Verknüpfung mit einer Diagnose, einer Prognose oder einem Ansprechen auf Behandlung eines Zustands oder einer Krankheit, wobei die Verknüpfung aus einem prädikativen Satz von Momenten vorausgesagt wird, welcher aus einem bestehenden Datensatz identifiziert wurde, der Datensatz Zellprofildaten umfasst, welche aus einer Vielzahl von Gewebeproben erzeugt wurden, welche einer Kohorte von Patienten entnommen wurden, welche eine Kommunalität aufweisen, der Datensatz weiter eine Verknüpfung der Zellprofildaten mit zumindest einem Stück von Metainformationen aufweist, welche eine Sichtfeldebenenbewertung oder Patientenebenenbewertung in Bezug auf die Kommunalität beinhalten, die Verknüpfung eine Verknüpfung zwischen beobachteten ersten Momenten und einer Sichtfeldebenenbewertung oder einer Patientenebenenbewertung beschreibt, welche durch Einpassen eines Klassifizierungsmodells mit der Bewertung als Ergebnis und ersten Momenten als Prädiktoren abgeleitet wird, und die Bewertung eine Diagnose, eine Prognose oder ein Ansprechen auf Behandlung eines Zustands oder einer Krankheit anzeigt.

2. System nach Anspruch 1, weiter eine Kamera zur digitalen Bilderfassung der zumindest einen Gewebeprobe von dem Patienten umfassend;
   wobei optional der zumindest eine Prozessor weiter Code ausführt, welcher den zumindest einen Prozessor veranlasst, den Schritt des Segmentierens individueller Zelle der zumindest einen Gewebeprobe von dem Patienten in Kammern auf Basis der erzeugten Bilder, welche den Ausdruck jedes der Vielzahl von Biomarkern erfassen, auszuführen.

3. System nach Anspruch 1 oder wobei der zumindest eine Prozessor weiter Code ausführt, welcher den zumindest einen Prozessor veranlasst, die folgenden Schritte auszuführen:

   Berechnen zumindest zweier Zellmerkmale auf Basis des Ausdrucks der Zelle jedes der Vielzahl von nicht morphologischen Biomarkern; und
   Berechnen eines ersten Moments für jedes der zumindest zwei Zellmerkmale für jedes des zumindest einen Sichtfelds.

4. System nach Anspruch 1, wobei der zumindest eine Prozessor weiter Code ausführt, welcher den zumindest einen Prozessor veranlasst, die folgenden Schritte auszuführen:

   Berechnen zumindest dreier Zellmerkmale auf Basis des Ausdrucks der Zelle jedes der Vielzahl von nicht morphologischen Biomarkern; und
   Berechnen eines ersten Moments für jedes der zumindest drei Zellmerkmale für jedes Vielzahl von Sichtfeldern.

5. System nach Anspruch 1, wobei der zumindest eine Prozessor weiter Code ausführt, welcher den zumindest einen Prozessor veranlasst, die folgenden Schritte auszuführen:

Berechnen eines zweiten Moments für jedes der Vielzahl von Sichtfeldern aus jedem des zumindest einen Zellmerkmals; und

Untersuchen der berechneten ersten und zweiten Momente für eine Verknüpfung mit einer Diagnose, einer Prognose oder ein Ansprechen auf Behandlung eines Zustands oder einer Krankheit.

6. System nach Anspruch 5, wobei der zumindest eine Prozessor weiter Code ausführt, welcher den zumindest einen Prozessor veranlasst, die folgenden Schritte auszuführen:

Berechnen eines dritten Moments für jedes der Vielzahl von Sichtfeldern aus jedem des zumindest einen Zellmerkmals; und

Untersuchen der berechneten ersten, zweiten und dritten Momente für eine Verknüpfung mit einer Diagnose, einer Prognose oder ein Ansprechen auf Behandlung eines Zustands oder einer Krankheit.

7. System nach Anspruch 6, wobei die Momente Mittel, Standardabweichung und Schiefe umfassen.

8. System nach Anspruch 1, wobei die Verknüpfung mit der Diagnose oder der Prognose der Krankheit einen Zellgrad oder eine Überlebensdauer umfasst.

## Revendications

1. Système d'analyse de caractéristiques de tissu sur la base de données d'image générées au moyen de techniques de coloration/décoloration de multiplexage, le système comprenant :
au moins un processeur pour exécuter un code qui amène l'au moins un processeur à effectuer les étapes suivantes :

la génération d'images d'une pluralité de champs de vision colorés, dans lequel les images générées illustrent l'expression d'une pluralité de biomarqueurs dans des cellules individuelles qui font partie d'un plus grand échantillon cellulaire, dans lequel le plus grand échantillon cellulaire comprend au moins un échantillon de tissu d'un patient,
un dispositif de stockage pour stocker des données de profil cellulaire comprenant les images générées capturant l'expression d'une pluralité de biomarqueurs par l'au moins un échantillon de tissu d'un patient par rapport à une pluralité de champs de vision dans lesquels des cellules individuelles sont délimitées et segmentées en compartiments, et
l'au moins un processeur étant adapté pour exécuter un code qui amène en outre l'au moins un processeur à effectuer les étapes suivantes :

le calcul d'au moins une caractéristique cellulaire sur la base de l'expression cellulaire de chacun de la pluralité de biomarqueurs ;
le calcul, pour chacun de la pluralité de champs de vision et pour chacune de l'au moins une caractéristique cellulaire, d'un premier moment de la caractéristique cellulaire pour les cellules dans le champ de vision, dans lequel le premier moment est une sélection de ou une moyenne, d'un écart type et d'une asymétrie ; et
l'examen des premiers moments calculés pour une association avec un diagnostic, un pronostic ou une réponse au traitement d'une condition ou d'une maladie, dans lequel l'association est prédite à partir d'un ensemble prédictif de moments qui a été identifié à partir d'un ensemble de données existant, l'ensemble de données comprend des données de profil cellulaire générées à partir d'une pluralité d'échantillons de tissu prélevés à partir d'une cohorte de patients présentant un point commun, l'ensemble de données comprend en outre une association des données de profil cellulaire à au moins un élément de méta-informations incluant une évaluation au niveau champ de vision ou une évaluation au niveau patient relative au point commun, l'association décrit une association entre des premiers moments observés et une évaluation au niveau champ de vision ou une évaluation au niveau patient qui est obtenue en ajustant un modèle de classification avec l'évaluation comme un résultat et des premiers moments comme des prédicteurs, et l'évaluation est indicative d'un diagnostic, d'un pronostic ou d'une réponse au traitement d'une condition ou d'une maladie.

2. Système selon la revendication 1, comprenant en outre une caméra pour l'imagerie numérique de l'au moins un échantillon de tissu du patient ;

éventuellement dans lequel l'au moins un processeur exécute en outre un code qui amène l'au moins un processeur à effectuer l'étape de segmentation de cellules individuelles de l'au moins un échantillon de tissu du patient en compartiments sur la base des images générées capturant l'expression de chacun de la pluralité de biomarqueurs.

3. Système selon la revendication 1 ou dans lequel l'au moins un processeur exécute en outre un code qui amène l'au moins un processeur à effectuer les étapes suivantes :

le calcul d'au moins deux caractéristiques cellulaires sur la base de l'expression cellulaire de chacun de la pluralité de biomarqueurs non morphologiques ; et
le calcul d'un premier moment pour chacune des au moins deux caractéristiques cellulaires pour chacun de l'au moins un champ de vision.

4. Système selon la revendication 1, dans lequel l'au moins un processeur exécute en outre un code qui amène l'au moins un processeur à effectuer les étapes suivantes :

le calcul d'au moins trois caractéristiques cellulaires sur la base de l'expression cellulaire de chacun de la pluralité de biomarqueurs non morphologiques ; et
le calcul d'un premier moment pour chacune des au moins trois caractéristiques cellulaires pour chacun de la pluralité de champs de vision.

5. Système selon la revendication 1, dans lequel l'au moins un processeur exécute en outre un code qui amène l'au moins un processeur à effectuer les étapes suivantes :

le calcul d'un deuxième moment pour chacun de la pluralité de champs de vision à partir de chacune de l'au moins une caractéristique cellulaire ; et
l'examen des premier et deuxième moments calculés pour une association avec un diagnostic, un pronostic ou une réponse au traitement d'une condition ou d'une maladie.

6. Système selon la revendication 5, dans lequel l'au moins un processeur exécute en outre un code qui amène l'au moins un processeur à effectuer les étapes suivantes :

le calcul d'un troisième moment pour chacun de la pluralité de champs de vision à partir de chacune de l'au moins une caractéristique cellulaire ; et
l'examen des premier, deuxième et troisième moments calculés pour une association avec un diagnostic, un pronostic ou une réponse au traitement d'une condition ou d'une maladie.

7. Système selon la revendication 6, dans lequel les moments comprennent une moyenne, un écart type et une asymétrie.

8. Système selon la revendication 1, dans lequel l'association avec le diagnostic ou le pronostic de la maladie comprend un grade cellulaire ou un temps de survie.

Computing
device    <u>100</u>

Processor <u>102</u>

Core(s)    <u>104</u>

Visual diaplay
device    <u>122</u>

User
interface    <u>124</u>

Device
storage    <u>116</u>

Operating
system    <u>118</u>

Analysis
code    <u>150</u>

Memory    <u>106</u>

Multi-point
touch    <u>108</u>
interface

Pointing
Device    <u>110</u>

Network
interface    <u>112</u>

Network
device    <u>126</u>

Virtual
Machine    <u>114</u>

Processor <u>102</u>

Core(s)    <u>104</u>

## FIG. 1

200

Generate multiple sets of clusters from
data set      220

Observe proportion of cells assigned to
each cluster within multiple sets      230

Examine observed proportions for an
association      240

Select predictive set of clusters      250

## FIG. 2

300

| Identify cluster | 320 |

↓

| Create montage of cell | 330 |

↓

| Display montage of cell | 340 |

# FIG. 3

400

| Assign each cell to a cluster | 420 |

↓

| Observe proportion of cells assigned to each cluster | 430 |

↓

| Examine observed proportions for an association | 440 |

# FIG. 4

500

| Calculating cell features based on biomarker expression | 520 |
|---|---|

↓

| Calculating moments from the cell features | 530 |
|---|---|

↓

| Examining the calculated moments for an association | 540 |
|---|---|

↓

| Selecting a predective combination of moments | 550 |
|---|---|

## FIG. 5

600

| Calculating at least one cell feature | 620 |
|---|---|

↓

| Calculating a moment for each cell feature | 630 |
|---|---|

↓

| Examining the calculated moments for an association | 640 |
|---|---|

## FIG. 6

FIG. 7

FIG. 8

FIG. 9

FIG. 10

FIG. 11

FIG. 12

FIG. 13

FIG. 14

FIG. 15

FIG. 16

FIG. 17

FIG. 18

FIG. 19

FIG. 20

FIG. 21

FIG. 22

FIG. 23

FIG. 24

FIG. 25

FIG. 26

FIG. 27

FIG. 28

FIG. 29

FIG. 30

FIG. 31

FIG. 32

FIG. 33

FIG. 34

FIG. 35

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 20110091081 A

- US 20110091091 A

**Non-patent literature cited in the description**

- **L. BREIMAN.** Random Forests. *Machine Learning,* 2001, vol. 45 (1), 5-32
- **H. ISHWARAN et al.** Random Survival Forests. *Ann. App. Statist.,* 2008, vol. 2, 841-860

- **HARRELL, F.E. et al.** Evaluating the Yield of Medical Tests. *J. Amer. Med. Assoc.,* 1982, vol. 247, 2543-2546